# EUROPEAN PATENT APPLICATION

(11) **EP 3 677 259 A1**
(43) Date of publication of application: **08.07.2020**
(21) Application number: 19150606.2
(22) Date of filing: 07.01.2019
(51) Int. Cl.: A61K 31/121, A61K 45/06, A61K 31/137, A61K 31/16, A61K 31/192, A61K 31/4045, A61K 31/405, A61K 31/426, A61K 31/473, A61K 31/662, A61P 9/10, A61P 9/00

(54) **USE OF ARACHIDONYL TRIFLUOROMETHYL KETONE**

(71) Applicant: Mosaiques Diagnostics And Therapeutics AG, 30659 Hannover (DE)
(72) Inventor: The designation of the inventor has not yet been filed
(74) Representative: Bohmann, Armin K.

(57) **Abstract**

The present invention is related to an inhibitor of cytosolic human phospholipase A2 (cPLA2) activity for use in the treatment of a subject suffering from or being at risk of suffering from a disease caused by or associated with vascular calcification.

SEQUENCE LISTING

<110>  Mosaiques Diagnostics and Therapeutics AG

<120>  Use of arachidonyl trifluoromethyl ketone

<130>  M 10139 EP

<160>  4

<170>  PatentIn version 3.5

<210>  1
<211>  749
<212>  PRT
<213>  Homo sapiens


<220>
<221>  misc_feature
<223>  Isoform 1 of cytoplasmic PLA2

<400>  1

Met Ser Phe Ile Asp Pro Tyr Gln His Ile Ile Val Glu His Gln Tyr
1               5                   10                  15


Ser His Lys Phe Thr Val Val Val Leu Arg Ala Thr Lys Val Thr Lys
            20                  25                  30


Gly Ala Phe Gly Asp Met Leu Asp Thr Pro Asp Pro Tyr Val Glu Leu
        35                  40                  45


Phe Ile Ser Thr Thr Pro Asp Ser Arg Lys Arg Thr Arg His Phe Asn
    50                  55                  60


Asn Asp Ile Asn Pro Val Trp Asn Glu Thr Phe Glu Phe Ile Leu Asp
65                  70                  75                  80


Pro Asn Gln Glu Asn Val Leu Glu Ile Thr Leu Met Asp Ala Asn Tyr
                85                  90                  95


Val Met Asp Glu Thr Leu Gly Thr Ala Thr Phe Thr Val Ser Ser Met
            100                 105                 110


Lys Val Gly Glu Lys Lys Glu Val Pro Phe Ile Phe Asn Gln Val Thr
        115                 120                 125


Glu Met Val Leu Glu Met Ser Leu Glu Val Cys Ser Cys Pro Asp Leu
    130                 135                 140


Arg Phe Ser Met Ala Leu Cys Asp Gln Glu Lys Thr Phe Arg Gln Gln
145                 150                 155                 160

Arg Lys Glu His Ile Arg Glu Ser Met Lys Lys Leu Leu Gly Pro Lys
                165                 170                 175

Asn Ser Glu Gly Leu His Ser Ala Arg Asp Val Pro Val Val Ala Ile
            180                 185                 190

Leu Gly Ser Gly Gly Gly Phe Arg Ala Met Val Gly Phe Ser Gly Val
        195                 200                 205

Met Lys Ala Leu Tyr Glu Ser Gly Ile Leu Asp Cys Ala Thr Tyr Val
    210                 215                 220

Ala Gly Leu Ser Gly Ser Thr Trp Tyr Met Ser Thr Leu Tyr Ser His
225                 230                 235                 240

Pro Asp Phe Pro Glu Lys Gly Pro Glu Glu Ile Asn Glu Glu Leu Met
                245                 250                 255

Lys Asn Val Ser His Asn Pro Leu Leu Leu Leu Thr Pro Gln Lys Val
            260                 265                 270

Lys Arg Tyr Val Glu Ser Leu Trp Lys Lys Lys Ser Ser Gly Gln Pro
        275                 280                 285

Val Thr Phe Thr Asp Ile Phe Gly Met Leu Ile Gly Glu Thr Leu Ile
    290                 295                 300

His Asn Arg Met Asn Thr Thr Leu Ser Ser Leu Lys Glu Lys Val Asn
305                 310                 315                 320

Thr Ala Gln Cys Pro Leu Pro Leu Phe Thr Cys Leu His Val Lys Pro
                325                 330                 335

Asp Val Ser Glu Leu Met Phe Ala Asp Trp Val Glu Phe Ser Pro Tyr
            340                 345                 350

Glu Ile Gly Met Ala Lys Tyr Gly Thr Phe Met Ala Pro Asp Leu Phe
        355                 360                 365

Gly Ser Lys Phe Phe Met Gly Thr Val Val Lys Lys Tyr Glu Glu Asn
    370                 375                 380

Pro Leu His Phe Leu Met Gly Val Trp Gly Ser Ala Phe Ser Ile Leu
385                 390                 395                 400

Phe Asn Arg Val Leu Gly Val Ser Gly Ser Gln Ser Arg Gly Ser Thr
                405                 410                 415

Met Glu Glu Glu Leu Glu Asn Ile Thr Thr Lys His Ile Val Ser Asn
            420                 425                 430

Asp Ser Ser Asp Ser Asp Asp Glu Ser His Glu Pro Lys Gly Thr Glu
        435                 440                 445

Asn Glu Asp Ala Gly Ser Asp Tyr Gln Ser Asp Asn Gln Ala Ser Trp
    450                 455                 460

Ile His Arg Met Ile Met Ala Leu Val Ser Asp Ser Ala Leu Phe Asn
465                 470                 475                 480

Thr Arg Glu Gly Arg Ala Gly Lys Val His Asn Phe Met Leu Gly Leu
                485                 490                 495

Asn Leu Asn Thr Ser Tyr Pro Leu Ser Pro Leu Ser Asp Phe Ala Thr
            500                 505                 510

Gln Asp Ser Phe Asp Asp Asp Glu Leu Asp Ala Ala Val Ala Asp Pro
        515                 520                 525

Asp Glu Phe Glu Arg Ile Tyr Glu Pro Leu Asp Val Lys Ser Lys Lys
    530                 535                 540

Ile His Val Val Asp Ser Gly Leu Thr Phe Asn Leu Pro Tyr Pro Leu
545                 550                 555                 560

Ile Leu Arg Pro Gln Arg Gly Val Asp Leu Ile Ile Ser Phe Asp Phe
                565                 570                 575

Ser Ala Arg Pro Ser Asp Ser Ser Pro Pro Phe Lys Glu Leu Leu Leu
            580                 585                 590

Ala Glu Lys Trp Ala Lys Met Asn Lys Leu Pro Phe Pro Lys Ile Asp
        595                 600                 605

Pro Tyr Val Phe Asp Arg Glu Gly Leu Lys Glu Cys Tyr Val Phe Lys
    610                 615                 620

Pro Lys Asn Pro Asp Met Glu Lys Asp Cys Pro Thr Ile Ile His Phe
625                 630                 635                 640

Val Leu Ala Asn Ile Asn Phe Arg Lys Tyr Lys Ala Pro Gly Val Pro
                645                 650                 655

Arg Glu Thr Glu Glu Glu Lys Glu Ile Ala Asp Phe Asp Ile Phe Asp

            660                 665                 670

Asp Pro Glu Ser Pro Phe Ser Thr Phe Asn Phe Gln Tyr Pro Asn Gln
        675                 680                 685

Ala Phe Lys Arg Leu His Asp Leu Met His Phe Asn Thr Leu Asn Asn
    690                 695                 700

Ile Asp Val Ile Lys Glu Ala Met Val Glu Ser Ile Glu Tyr Arg Arg
705                 710                 715                 720

Gln Asn Pro Ser Arg Cys Ser Val Ser Leu Ser Asn Val Glu Ala Arg
                725                 730                 735

Arg Phe Phe Asn Lys Glu Phe Leu Ser Lys Pro Lys Ala
            740                 745

<210> 2
<211> 689
<212> PRT
<213> Homo sapiens

<220>
<221> misc_feature
<223> Isoform 2 of cytoplasmic PLA2

<400> 2

Met Ser Phe Ile Asp Pro Tyr Gln His Ile Ile Val Glu His Gln Tyr
1               5                   10                  15

Ser His Lys Phe Thr Val Val Val Leu Arg Ala Thr Lys Val Thr Lys
            20                  25                  30

Gly Ala Phe Gly Asp Met Leu Asp Thr Pro Asp Pro Tyr Val Glu Leu
        35                  40                  45

Phe Ile Ser Thr Thr Pro Asp Ser Arg Lys Arg Thr Arg His Phe Asn
    50                  55                  60

Asn Asp Ile Asn Pro Val Trp Asn Glu Thr Phe Glu Phe Ile Leu Asp
65                  70                  75                  80

Pro Asn Gln Glu Asn Val Leu Glu Ile Thr Leu Met Asp Ala Asn Tyr
                85                  90                  95

Val Met Asp Glu Thr Leu Gly Thr Ala Thr Phe Thr Val Ser Ser Met
            100                 105                 110

Lys Val Gly Glu Lys Lys Glu Val Pro Phe Ile Phe Asn Gln Val Pro
        115                 120                 125

Val Val Ala Ile Leu Gly Ser Gly Gly Gly Phe Arg Ala Met Val Gly
    130                 135                 140

Phe Ser Gly Val Met Lys Ala Leu Tyr Glu Ser Gly Ile Leu Asp Cys
145                 150                 155                 160

Ala Thr Tyr Val Ala Gly Leu Ser Gly Ser Thr Trp Tyr Met Ser Thr
                165                 170                 175

Leu Tyr Ser His Pro Asp Phe Pro Glu Lys Gly Pro Glu Glu Ile Asn
            180                 185                 190

Glu Glu Leu Met Lys Asn Val Ser His Asn Pro Leu Leu Leu Leu Thr
        195                 200                 205

Pro Gln Lys Val Lys Arg Tyr Val Glu Ser Leu Trp Lys Lys Lys Ser
    210                 215                 220

Ser Gly Gln Pro Val Thr Phe Thr Asp Ile Phe Gly Met Leu Ile Gly
225                 230                 235                 240

Glu Thr Leu Ile His Asn Arg Met Asn Thr Thr Leu Ser Ser Leu Lys
                245                 250                 255

Glu Lys Val Asn Thr Ala Gln Cys Pro Leu Pro Leu Phe Thr Cys Leu
            260                 265                 270

His Val Lys Pro Asp Val Ser Glu Leu Met Phe Ala Asp Trp Val Glu
        275                 280                 285

Phe Ser Pro Tyr Glu Ile Gly Met Ala Lys Tyr Gly Thr Phe Met Ala
    290                 295                 300

Pro Asp Leu Phe Gly Ser Lys Phe Phe Met Gly Thr Val Val Lys Lys
305                 310                 315                 320

Tyr Glu Glu Asn Pro Leu His Phe Leu Met Gly Val Trp Gly Ser Ala
                325                 330                 335

Phe Ser Ile Leu Phe Asn Arg Val Leu Gly Val Ser Gly Ser Gln Ser
            340                 345                 350

Arg Gly Ser Thr Met Glu Glu Glu Leu Glu Asn Ile Thr Thr Lys His
        355                 360                 365

Ile Val Ser Asn Asp Ser Ser Asp Ser Asp Asp Glu Ser His Glu Pro
    370                 375                 380

Lys Gly Thr Glu Asn Glu Asp Ala Gly Ser Asp Tyr Gln Ser Asp Asn
385                 390                 395                 400

Gln Ala Ser Trp Ile His Arg Met Ile Met Ala Leu Val Ser Asp Ser
                405                 410                 415

Ala Leu Phe Asn Thr Arg Glu Gly Arg Ala Gly Lys Val His Asn Phe
            420                 425                 430

Met Leu Gly Leu Asn Leu Asn Thr Ser Tyr Pro Leu Ser Pro Leu Ser
        435                 440                 445

Asp Phe Ala Thr Gln Asp Ser Phe Asp Asp Asp Glu Leu Asp Ala Ala
    450                 455                 460

Val Ala Asp Pro Asp Glu Phe Glu Arg Ile Tyr Glu Pro Leu Asp Val
465                 470                 475                 480

Lys Ser Lys Lys Ile His Val Val Asp Ser Gly Leu Thr Phe Asn Leu
                485                 490                 495

Pro Tyr Pro Leu Ile Leu Arg Pro Gln Arg Gly Val Asp Leu Ile Ile
            500                 505                 510

Ser Phe Asp Phe Ser Ala Arg Pro Ser Asp Ser Ser Pro Pro Phe Lys
        515                 520                 525

Glu Leu Leu Leu Ala Glu Lys Trp Ala Lys Met Asn Lys Leu Pro Phe
    530                 535                 540

Pro Lys Ile Asp Pro Tyr Val Phe Asp Arg Glu Gly Leu Lys Glu Cys
545                 550                 555                 560

Tyr Val Phe Lys Pro Lys Asn Pro Asp Met Glu Lys Asp Cys Pro Thr
                565                 570                 575

Ile Ile His Phe Val Leu Ala Asn Ile Asn Phe Arg Lys Tyr Lys Ala
            580                 585                 590

Pro Gly Val Pro Arg Glu Thr Glu Glu Glu Lys Glu Ile Ala Asp Phe
        595                 600                 605

Asp Ile Phe Asp Asp Pro Glu Ser Pro Phe Ser Thr Phe Asn Phe Gln
    610                 615                 620

Tyr Pro Asn Gln Ala Phe Lys Arg Leu His Asp Leu Met His Phe Asn
625                 630                 635                 640

Thr Leu Asn Asn Ile Asp Val Ile Lys Glu Ala Met Val Glu Ser Ile
                645                 650                 655

Glu Tyr Arg Arg Gln Asn Pro Ser Arg Cys Ser Val Ser Leu Ser Asn
            660                 665                 670

Val Glu Ala Arg Arg Phe Phe Asn Lys Glu Phe Leu Ser Lys Pro Lys
        675                 680                 685

Ala

<210>  3
<211>  21
<212>  DNA
<213>  Artificial Sequence

<220>
<223>  Synthetic

<220>
<221>  misc_feature
<222>  (20)..(21)
<223>  Deoxyribonucleotide

<400>  3
uguauugaga uucaagccca g                                                21

<210>  4
<211>  21
<212>  DNA
<213>  Artificial Sequence

<220>
<223>  Synthetic

<220>
<221>  misc_feature
<222>  (20)..(21)
<223>  Deoxyribonucleotide

<400>  4
gggcuugaau cucaauacat t                                                21

## Description

The present invention is related to an inhibitor of cytosolic human phospholipase A2 (cPLA2) activity for use in the treatment of a disease, an inhibitor of cytosolic human phospholipase A2 (cPLA2) activity for use in therapy, wherein therapy reverts a molecular phenotype of a disease, use of an inhibitor of cytosolic human phospholipase A2 (cPLA2) activity ketone for the manufacture of a medicament for the treatment of a disease, use of an inhibitor of cytosolic human phospholipase A2 (cPLA2) activity for the manufacture of a medicament for use in therapy, wherein therapy reverts a molecular phenotype of a disease, a method for the treatment of a disease associated with vascular calcification, and a method for the treatment of a molecular phenotype of cardiovascular disease.

Atherosclerotic cardiovascular diseases including coronary artery disease (CAD) are directly or indirectly the leading cause of morbidity and mortality worldwide (Lopez AD et al., Lancet. 2006 May 27;367(9524):1747-57). Overall, 40% of annual deaths in Europe are caused by CAD (Ferreira-Gonzalez I, Rev. Esp. Cardiol (Engl. Ed), 2014 Feb;67(2):139-44). Despite decline in CAD-related mortality due to better prevention and invasive treatment, the prevalence of CAD is still on the rise in part due to increased longevity and increased survival rates of myocardial infarctions (Gaziano TA et al., Curr Probl Cardiol. 2010 Feb;35(2):72-115). The annual cost of CAD in Europe is estimated at 49 billion euros of which approximately 48% is due to direct health care costs (PMID:23487016). The socio-economic burden of CAD is therefore obvious and innovative methods that will improve its prevention and management are urgently needed.

The pathophysiology of CAD is complex and diverse due to interactions between multiple features. It is generally accepted that genetic factors and predisposing conditions contribute to development of CAD in a complex combination with life style factors including exercise, nutrition, smoking and drinking (Ross R, N Engl J Med. 1999 Jan 14;340(2):115-26. PMID:9887164). Risk factors such as smoking, diabetes, hypertension and dyslipidaemia have been identified, and even more, common molecular features underlying the heterogeneous CAD phenotypes have been established, including oxidative stress, inflammation and extracellular matrix (ECM) remodelling. Due to the complexity of the disease, deciphering relevant molecular mechanisms based on single biomarkers remains challenging.

Although cardiovascular diseases have become a global burden and their prevalence is on the rise, the development of new drugs has been moderate over the years (Lagassé HA et al., F1000Res. 2017 Feb 7;6:113.). These drugs included for example lipid-lowering drugs alirocumab and evolocumab and sebelipase alfa, a drug to treat lysosomal acid lipase deficiency. Furthermore, both alirocumab and evolocumab have similar mode of action and target the human anti-propotein convertase substilisin/kexin type 9 (PCSK9) (Lagassé et al., supra). This stunted growth in the development of innovative compounds in the cardiovascular department is due to the moderate understanding of pathophysiological mechanisms associated with the disease. It is therefore obvious that the need to develop novel therapy for the treatment of CAD is pressing.

The current management of CAD relies on lifestyle modification, reduction of risk factors, pharmacological therapies such as cholesterol lowering drugs, antihypertensive drugs, and surgical interventions including coronary artery bypass grafting (CABG) and percutaneous coronary interventions (PCI) which are efficient in relieving symptoms and preventing complications (e.g. myocardial infarction and death) (Montalescot G et al., Eur Heart J. 2013 Oct;34(38):2949-3003) rather than dealing with the causative pathological mechanisms. Thus, there is a need to develop novel pharmacological therapies to improve the management of CAD given that coronary revascularization is invasive.

The problem underlying the present invention is the provision of a means for the treatment and/or prevention of a disease associated with vascular calcification. A further problem underlying the present invention is the provision of a means for reverting a molecular phenotype of a cardiovascular disease. Another problem underlying the present invention is the provision of a means for the treatment of a disease, wherein the treatment reverts a molecular phenotype of a cardiovascular disease. Still another problem underlying the present invention is the provision of a method for the treatment of a subject suffering from or being at risk of suffering from a disease associated with vascular calcification. Finally, a problem underlying the present invention is the provision of a method for reversing a molecule phenotype of a cardiovascular disease.

These and other problems are solved by the subject matter of the attached independent claims; preferred embodiments may be taken from the attached dependent claims.

More specifically, the problem underlying the present invention is solved in a first aspect, which is also the first embodiment of the first aspect, by an inhibitor of cytosolic human phospholipase A2 (cPLA2) activity, preferably arachidonyl trifluoromethyl ketone (AACOCF3) of formula (I) 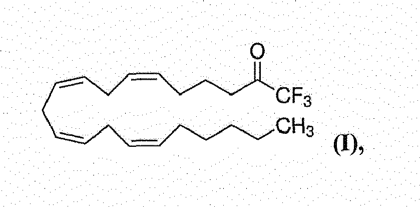 for use in the treatment of a subject suffering from or being at risk of suffering from a disease associated with vascular calcification.

More specifically, the problem underlying the present invention is solved in a second aspect by an inhibitor of cytosolic human phospholipase A2 (cPLA2) activity, preferably arachidonyl trifluoromethyl ketone (AACOCF3) of formula (I) 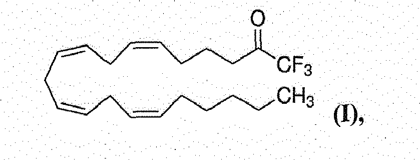 for use in therapy, wherein therapy reverts the molecular phenotype of a cardiovascular disease.

In a second embodiment of the first aspect, which is an embodiment of the first aspect, vascular calcification is mediated by vascular smooth muscle cells.

In a third embodiment of the first aspect, which is also an embodiment of the first and the second embodiment of the first aspect, vascular smooth muscle cells are re-modelled during calcification.

In a fourth embodiment of the first aspect, which is also an embodiment of the first, second and third embodiment of the first aspect, vascular calcification results from osteochondrogenic transdifferentiation.

In a fifth embodiment of the first aspect which is also an embodiment of the first, second, third and fourth embodiment of the first aspect, and in a second embodiment of the second aspect which is also an embodiment of the first embodiment of the second aspect, AACOCF3 inhibits cPLA2 activity.

In a sixth embodiment of the first aspect which is also an embodiment of the first, second, third, fourth and fifth embodiment of the first aspect, the disease is a cardiovascular disease.

In a seventh embodiment of the first aspect which is also an embodiment of the first, second, third, fourth, fifth and sixth embodiment of the first aspect, and in a fifth embodiment of the second aspect which is also an embodiment of the first, second, third and fourth embodiment of the second aspect, the cardiovascular disease is selected from the list comprising CAD, stroke, heart failure, hypertension, angina pectoris, myocardial infarction, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis (see, in particular as to the link between cPLA2 and cardiovascular disease, Elinder et al. Expression of phospholipase A2 isoforms in human normal and atherosclerotic arterial wall. Arterioscler Thromb Vasc Biol. 1997 Oct;17(10):2257-63; and Hartiala et al. Genetic contribution of the leukotriene pathway to coronary artery disease. Hum Genet. 2011 Jun;129(6):617-27; and for the impact of calcification of cardiovascular disease, see, New and Aikawa: Cardiovascular calcification: an inflammatory disease. Circ J. 2011;75(6):1305-13; and Harper et al. Vascular calcification in type-2 diabetes and cardiovascular disease: Integrative roles for OPG, RANKL and TRAIL. Vascul Pharmacol. 2016 Jul;82:30-40).

In an eighth embodiment of the first aspect which is also an embodiment of the first, second, third, fourth, fifth, sixth and seventh embodiment of the first aspect, and in a sixth embodiment of the second aspect which is also an embodiment of the first, second, third, fourth and fifth embodiment of the second aspect, the cardiovascular disease is an atherosclerotic cardiovascular disease preferably selected from the group comprising coronary artery disease, stroke, peripheral artery disease, and chronic kidney disease.

In a ninth embodiment of the first aspect which is also an embodiment of the first, second, third, fourth, fifth, sixth, seventh and eighth embodiment of the first aspect, and in a seventh embodiment of the second aspect which is also an embodiment of the first, second, third, fourth, fifth and sixth embodiment of the second aspect, the disease is hypertension.

In a tenth embodiment of the first aspect which is also an embodiment of the first, second, third, fourth, fifth, sixth, seventh, eighth and ninth embodiment of the first aspect, and in an eighth embodiment of the second aspect which is also an embodiment of the first, second, third, fourth, fifth, sixth and seventh embodiment of the second aspect, the disease is angina pectoris.

In an eleventh embodiment of the first aspect which is also an embodiment of the first, second, third, fourth, fifth, sixth, seventh, eighth, ninth and tenth embodiment of the first aspect, and in a ninth embodiment of the second aspect which is also an embodiment of the first, second, third, fourth, fifth, sixth, seventh and eighth embodiment of the second aspect, the disease is myocardial infarction.

In a twelfth embodiment of the first aspect which is also an embodiment of the first, second, third, fourth, fifth, sixth, seventh, eighth, ninth, tenth and eleventh embodiment of the first aspect, and in a tenth embodiment of the second aspect which is also an embodiment of the first, second, third, fourth, fifth, sixth, seventh, eighth and ninth embodiment of the second aspect, the disease is stroke.

In a 13^{th} embodiment of the first aspect which is also an embodiment of the first, second, third, fourth, fifth, sixth, seventh, eighth, ninth, tenth, eleventh and twelfth embodiment of the first aspect, and in an eleventh embodiment of the second aspect which is also an embodiment of the first, second, third, fourth, fifth, sixth, seventh, eighth, ninth and tenth embodiment of the second aspect, the disease is a chronic kidney disease, preferably diabetic kidney disease or nephrosclerosis.

In a 14^{th} embodiment of the first aspect which is also an embodiment of the first, second, third, fourth, fifth, sixth, seventh, eighth, ninth, tenth, eleventh, twelfth and 13^{th} embodiment of the first aspect, and in a twelfth embodiment of the second aspect which is also an embodiment of the first, second, third, fourth, fifth, sixth, seventh, eighth, ninth, tenth and eleventh embodiment of the second aspect, the disease is a chronic kidney disease, the subject shows at least one cardiovascular disease risk factor selected from the group comprising little exercise, unhealthy nutrition, smoking and drinking.

In a 15^{th} embodiment of the first aspect which is also an embodiment of the first, second, third, fourth, fifth, sixth, seventh, eighth, ninth, tenth, eleventh, twelfth, 13^{th} and 14^{th} embodiment of the first aspect, and in a 13^{th} embodiment of the second aspect which is also an embodiment of the first, second, third, fourth, fifth, sixth, seventh, eighth, ninth, tenth, eleventh and twelfth embodiment of the second aspect, the subject has a risk factor selected from the group comprising smoking, diabetes, hypertension and dyslipidaemia.

In a 16^{th} embodiment of the first aspect which is also an embodiment of the first, second, third, fourth, fifth, sixth, seventh, eighth, ninth, tenth, eleventh, twelfth, 13^{th}, 14^{th} and 15^{th} embodiment of the first aspect, and in a 14^{th} embodiment of the second aspect which is also an embodiment of the first, second, third, fourth, fifth, sixth, seventh, eighth, ninth, tenth, eleventh, twelfth and 13^{th} embodiment of the second aspect, the treatment further comprises a therapy selected from the group comprising a cholesterol lowering drug, an antihypertensive drug and surgical intervention, preferably coronary artery bypass grafting (CABG), percutaneous coronary intervention (PCI) and stenting of vasculature.

In a 17^{th} embodiment of the first aspect which is also an embodiment of the first, second, third, fourth, fifth, sixth, seventh, eighth, ninth, tenth, eleventh, twelfth, 13^{th}, 14^{th}, 15^{th} and 16^{th} embodiment of the first aspect, and in a 15^{th} embodiment of the second aspect which is also an embodiment of the first, second, third, fourth, fifth, sixth, seventh, eighth, ninth, tenth, eleventh, twelfth, 13^{th} and 14^{th} embodiment of the second aspect, the cholesterol lowering drug is selected from the group comprising a statin, a fibrate, niacin, and a bile acid sequestrant. In a preferred embodiment thereof, the cholesterol lowering drug is selected from the group comprising atorvastatin, pravastatin, fenofibrate, bezafibrate, vitamine B3, cholestagel and cholestyramine.

In an 18^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th} and 17^{th} embodiment of the first aspect, and in a 16^{th} embodiment of the second aspect which is also an embodiment of the first, second, third, fourth, fifth, sixth, seventh, eighth, ninth, tenth, eleventh, twelfth, 13^{th}, 14^{th} and 15^{th} embodiment of the second aspect, the antihypertensive drug is selected from the group comprising a diuretic, a calcium channel blocker, an ACE inhibitor, an angiotensin II receptor antagonist, an adrenergic receptor antagonist, a vasodilator, a renin inhibitor, an aldosterone receptor antagonist, an alpha-2 adrenergic receptor agonist, and an endothelin receptor blocker. This teaching is based on the finding that lowering blood pressure using antihypertensive drugs is beneficial in cardiovascular disease and significantly reduces vascular risk (see, e.g., Ettehad D et al., Lancet. 2016 Mar 5;387(10022):957-967). More specifically, the problem underlying the present invention is solved in a third aspect, which is also a first embodiment of the third aspect, by the use of an arachidonyl trifluoromethyl ketone (AACOCF3) of formula (I) 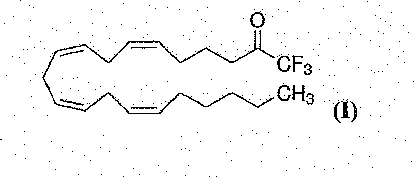 in the manufacture of a medicament, wherein the medicament is for use in the treatment of a subject suffering from or being at risk of suffering from a disease associated with or caused by vascular calcification.

More specifically, the problem underlying the present invention is solved in a fourth aspect, which is also a first embodiment of the fourth aspect, by the use of an arachidonyl trifluoromethyl ketone (AACOCF3) of formula (I) 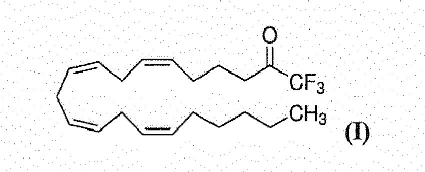 in the manufacture of a medicament, wherein the medicament is for reverting the molecular phenotype of a cardiovascular disease.

In a second embodiment of the third aspect which is also an embodiment of the first embodiment of the third aspect, and in a second embodiment of the fourth aspect which is also an embodiment of the first embodiment of the fourth aspect, AACOCF3 inhibits the activity of cPLA2.

In a third embodiment of the third aspect which is also an embodiment of the first and second embodiment of the third aspect, and in a third embodiment of the fourth aspect which is also an embodiment of the first and second embodiment of the fourth aspect, AACOCF3 inhibits migration of vascular smooth muscle cells.

In a fourth embodiment of the third aspect which is also an embodiment of the first, second and third embodiment of the third aspect, and in a fourth embodiment of the fourth aspect which is also an embodiment of the first, second and third embodiment of the fourth aspect, AACOCF3 inhibits migration of endothelial cells, preferably vascular endothelial cells.

In a fifth embodiment of the third aspect which is also an embodiment of the first, second, third and fourth embodiment of the third aspect, and in a fifth embodiment of the fourth aspect which is also an embodiment of the first, second, third and fourth embodiment of the fourth aspect, the medicament is for the treatment of a cardiovascular disease is selected from the list comprising CAD, stroke, heart failure, hypertension, angina pectoris, myocardial infarction, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis.

In a sixth embodiment of the third aspect which is also an embodiment of the first, second, third, fourth and fifth embodiment of the third aspect, and in a sixth embodiment of the fourth aspect which is also an embodiment of the first, second, third, fourth and fifth embodiment of the fourth aspect, the cardiovascular disease is an atherosclerotic cardiovascular disease preferably selected from the group comprising coronary artery disease, stroke, peripheral artery disease, and chronic kidney disease.

In a seventh embodiment of the third aspect which is also an embodiment of the first, second, third, fourth, fifth and sixth embodiment of the third aspect, and in a seventh embodiment of the fourth aspect which is also an embodiment of the first, second, third, fourth, fifth and sixth embodiment of the fourth aspect, the disease is a chronic kidney disease, preferably diabetic kidney disease or nephrosclerosis.

In an eighth embodiment of the third aspect which is also an embodiment of the first, second, third, fourth, fifth, sixth and seventh embodiment of the third aspect, and in an eighth embodiment of the fourth aspect which is also an embodiment of the first, second, third, fourth, fifth, sixth and seventh embodiment of the fourth aspect, the medicament is for use together with a therapy selected from the group comprising a cholesterol lowering drug, an antihypertensive drug and surgical intervention, preferably coronary artery bypass grafting (CABG), percutaneous coronary intervention (PCI) and stenting of vasculature.

In a ninth embodiment of the third aspect which is also an embodiment of the first, second, third, fourth, fifth, sixth, seventh and eighth embodiment of the third aspect, and in an ninth embodiment of the fourth aspect which is also an embodiment of the first, second, third, fourth, fifth, sixth, seventh and eighth embodiment of the fourth aspect, the medicament further comprises a second therapeutically active agent, wherein the second therapeutically active agent is preferably selected from the group comprising a cholesterol lowering drug and an antihypertensive drug.

More specifically, the problem underlying the present invention is solved in a fifth aspect which is also a first embodiment of the fifth aspect, by a method for the treatment of a subject suffering from or being at risk of suffering from a disease associated with vascular calcification, wherein the method comprises administering to the subject a therapeutically active amount of an arachidonyl trifluoromethyl ketone (AACOCF3) of formula (I)

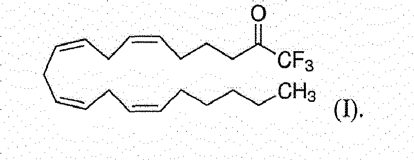

More specifically, the problem underlying the present invention is solved in a sixth aspect, which is also a first embodiment of the sixth as aspect, by a method for reversing the molecular phenotype of cardiovascular disease in a subject, wherein the method comprises administering to the subject arachidonyl trifluoromethyl ketone (AACOCF3) of formula (I)

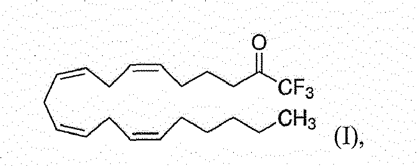

preferably a therapeutically effective amount of arachidonyl trifluoromethyl ketone (AACOCF3) of formula (I).

In a second embodiment of the fifth aspect which is also an embodiment of the first embodiment of the fifth aspect, and in a second embodiment of the sixth aspect which is also an embodiment of the first embodiment of the sixth aspect, AACOCF3 inhibits cPLA2 activity.

In a third embodiment of the fifth aspect which is also an embodiment of the first and second embodiment of the fifth aspect, and in a third embodiment of the sixth aspect which is also an embodiment of the first and second embodiment of the sixth aspect, AACOCF3 inhibits migration of vascular smooth muscle cells.

In a fourth embodiment of the fifth aspect which is also an embodiment of the first, second and third embodiment of the fifth aspect, and in a fourth embodiment of the sixth aspect which is also an embodiment of the first, second and third embodiment of the sixth aspect, AACOCF3 inhibits migration of endothelial cells, preferably vascular endothelial cells.

In a fifth embodiment of the fifth aspect which is also an embodiment of the first, second, third and fourth embodiment of the fifth aspect, and in a fifth embodiment of the sixth aspect which is also an embodiment of the first, second, third and fourth embodiment of the sixth aspect, the disease is a cardiovascular disease is selected from the list comprising CAD, stroke, heart failure, hypertension, angina pectoris, myocardial infarction, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis.

In a sixth embodiment of the fifth aspect which is also an embodiment of the first, second, third, fourth and fifth embodiment of the fifth aspect, and in a sixth embodiment of the sixth aspect which is also an embodiment of the first, second, third, fourth and fifth embodiment of the sixth aspect, the cardiovascular disease is an atherosclerotic cardiovascular disease preferably selected from the group comprising coronary artery disease, stroke, peripheral artery disease, and chronic kidney disease.

In a seventh embodiment of the fifth aspect which is also an embodiment of the first, second, third, fourth, fifth and sixth embodiment of the fifth aspect, and in a seventh embodiment of the sixth aspect which is also an embodiment of the first, second, third, fourth, fifth and sixth embodiment of the sixth aspect, the disease is a chronic kidney disease, preferably diabetic kidney disease or nephrosclerosis.

In an eighth embodiment of the fifth aspect which is also an embodiment of the first, second, third, fourth, fifth, sixth and seventh embodiment of the fifth aspect, and in an eighth embodiment of the sixth aspect which is also an embodiment of the first, second, third, fourth, fifth, sixth and seventh embodiment of the sixth aspect, the method comprises administering to the subject a therapy selected from the group comprising a cholesterol lowering drug, an antihypertensive drug and surgical intervention, preferably coronary artery bypass grafting (CABG), percutaneous coronary intervention (PCI) and stenting of vasculature.

In a ninth embodiment of the fifth aspect which is also an embodiment of the first, second, third, fourth, fifth, sixth, seventh and eighth embodiment of the fifth aspect, and in a ninth embodiment of the sixth aspect which is also an embodiment of the first, second, third, fourth, fifth, sixth, seventh and eighth embodiment of the sixth aspect, the method comprises administering a second therapeutically active agent, wherein the second therapeutically active agent is preferably selected from the group comprising a cholesterol lowering drug and an antihypertensive drug.

The present invention is based on the surprising finding that compound arachidonyl trifluoromethyl ketone which is of formula (I) 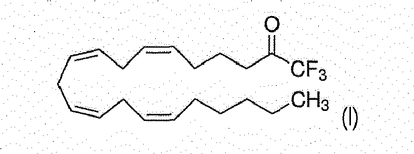 and which is also referred to as AACOCF3, is capable of affecting vascular calcification. Such vascular calcification is associated with, goes along with and/or causes various diseases, preferably cardiovascular diseases (see, e.g., Mathew RO et al., Kidney Int. 2017 Apr;91(4): 797-807; Sophie EP et al., Circulation Journal 75(6):1305-13 · May 2011; and Harper E et al., Vascular Pharmacology 82 · February 2016: 30-40) and chronic kidney disease (see, e.g., Mathew RO et al., Kidney Int. 2017 Apr;91(4): 797-807; Shroff R et al., JASN February 2013 vol. 24 no. 2 179-189).Vascluar calcification may involve or mediated by vascular smooth muscle cells which are preferably remodeled during the calcification process. Vascular calcification may be the result of osteochondrogenic transdifferentiation which may or may not involve remodeling of vascular smooth muscle cells (see, e.g., Shao JS et al., Arterioscler Thromb Vasc Biol. 2006 Jul;26(7):1423-30; and Kauffenstein G et al., Arteriosclerosis Thrombosis and Vascular Biology 34(5)) .

The present invention is further based on the surprising finding that compound arachidonyl trifluoromethyl ketone which is of formula (I) 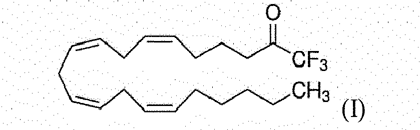 and which is also referred to as AACOCF3, is capable of reverting a phenotype of a cardiovascular disease and/or a phenotype of a disease which is associated with, goes along with and/or is caused by vascular calcification, preferably vascular calcification as disclosed and/or defined herein.

In an embodiment, and as preferably used herein, reverting a molecular phenotype means changing the molecular phenotype such that it is statistically changed compared to the molecular phenotype determined prior to the treatment, preferably treatment of the subject, with an arachidonyl trifluoromethyl ketone (AACOCF3) of formula (I)

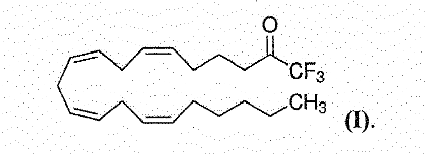

It will be appreciated by a person skilled in the art that factually each and any inhibitor of cPLA2 is suitable for use in the instant invention.

It is known in the art that cPLA2 may be either inhibited by inhibitors of cPLA2 binding stoichiometrically to cPLA12 or by inhibitors of cPLA2 which are nonspecific perturbants (Ramarao MK et al, Analytical Biochemistry 383 (2008), pp. 217-225). Both types of inhibitors are suitable for use in the practicing of the instant invention, i.e. for use in the treatment of a subject suffering from or being at risk of suffering from a disease caused by or associated with vascular calcification and for use in therapy, preferably in a therapy applied to a subject, wherein therapy reverts a molecular phenotype of a cardiovascular disease.

Possible inhibitors of cPLA2 are 2-oxoester as, for example, disclosed in Kokotou MG et al. (Nature, Scientific reports, 7: 7025 | DOI:10.1038/s41598-017-07330-5, published online 01 August 2017 and indicated in the following table

| Entry | No | Structure | GIVA cPLA₂ | | GVIA iPLA₂ | | GV sPLA₂ | ClogP |
|---|---|---|---|---|---|---|---|---|
| | | | % | X₁(50) | % Inhibition^{a} | X₁(50 | % | |
| 1 | 10e | 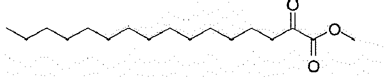 | 68.2 ± 2.7 | | 69.4 ± 12.2 | | 27.5 ± 0.9 | 6.51 |
| 2 | 12e | 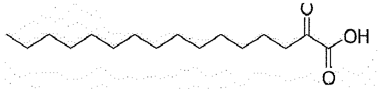 | 78.4 ± 3.5 | | <25 | | <25 | 5.63 |
| 3 | 17a | 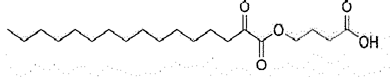 | >95 | 0.00008 ± 0.00001 | <25 | | <25 | 6.76 |
| 4 | 20 | 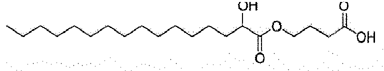 | <25 | | <25 | | 41.0 ± 0.2 | 6.82 |
| 5 | 17b | 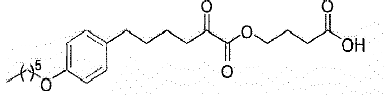 | >95 | 0.00289 ± 0.00043 | <25 | | 52.6 ± 5.3 | 5.46 |
| 6 | 16c | 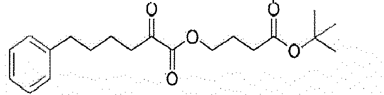 | <25 | | <25 | | <25 | 4.58 |
| 7 | 19 | 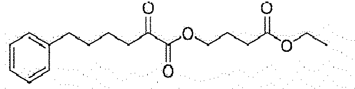 | 27.3 ± 4.8 | | <25 | | <25 | 3.87 |
| 8 | 17d | 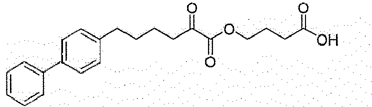 | >95 | 0.00068 ± 0.00007 | <25 | | <25 | 4.78 |
| 9 | 17e | 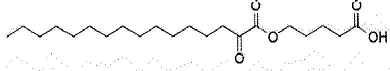 | > 95 | 0.00007 ± 0.00001 | 25 | | <25 | 6.68 |
| 10 | 17f | 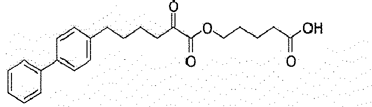 | > 95 | 0.000078 ± 0.00001 | 65 ± 3.4 | | <25 | 4.70 |
| 11 | 17g | 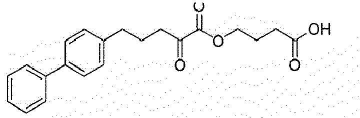 | > 95 | 0.0065 ± 0.002 | 84 ± 1.5 | | <25 | 4.25 |
| 12 | 17h | | > 95 | 0.0010 ± 0.0003 | 94 ± 1.4 | | <25 | 4.17 |
| 13 | 10a | 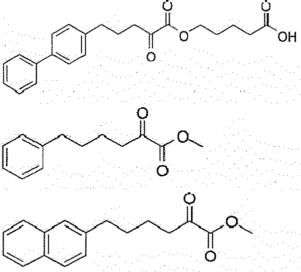 | <25 | | 72 ± 4 | | <25 | |
| 14 | 10b | | 55 ± 4.0 | | > 95 | 0.005 2 ± 0.000 | <25 | 3.81 |
| | | | | | | | | |

| Entry | No | Structure | GIVA cPLA₂ GVIA | | iPLA₂ | | GV sPLA₂ | ClogP |
|---|---|---|---|---|---|---|---|---|
| | | | % Inhibition^{a} | X₁(50) | % Inhibition^{a} | X₁(50 | % Inhibition^{a} | |
| 15 | 4 | 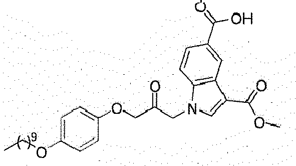 | >95 | 0.00008 ± 0.000005^{c} | | | | 8.50 |

Further inhibitors of cytosolic phospholipase A2 are the following ones:
Palmityl trifluoromethylketone, PTK, of the following formula 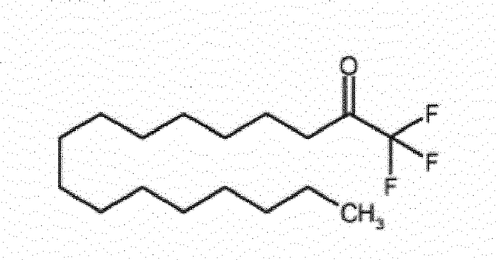 whereby PTK is an analog of palmitic acid in which the COOH group is replaced by trifluoromethyl ketone. PACOCF3 is an inhibitor of both Ca2+-dependent cytosolic cPLA2 (IC50=45 µM) and Ca2+-independent group VI iPLA2 (phospholipases A2 iPLA2, IC50=3.8 µM) (see, e.g., Kauser, K., et al. 1991. Circ. Res. 68: 1154-1163; Ackermann, E.J., et al. 1995. J. Biol. Chem. 27: 445-450; Lio, Y.C., et al. 1996. Biochim. Biophys. Acta. 1302: 55-60; and Jan, C.R., et al. 2000. Arch. Toxicol. 74: 447-451);
Fingolimod, FTY20, of the following formula

| **Strukturformel** | | |
|---|---|---|
| 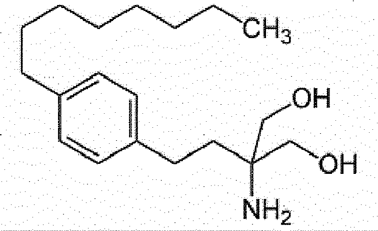 | | |

| **Allgemeines** | | |
|---|---|---|
| Freiname | Fingolimod | |
| | • | FTY-720 |
| Andere Namen | • | 2-Amino-2-(2-(4-octylphenyl)ethyl)propan-1,3-diol |
| Summenformel | C₁₉H₃₃NO₂ | |

whereby this immunosuppressant drug FTY720 inhibits cytosolic phospholipase A2 independently of sphingosine-1-phosphate receptors (see, e.g., Payne, Shawn G.; Oskeritzian, Carole A.; Griffiths, Plachael et al.;BLOOD Volume: 109 Issue: 3 Pages: 1077-1085) which is also referred to as 2-amino-2-(2-(4-octylphenyl)ethyl)propane-1,3-diol;
methyl arachidonyl fluorophosphonat (MAFP) (methylphosphonofluoridic acid 5,8,11,14-eicosatetraenyl ester) of the following formula 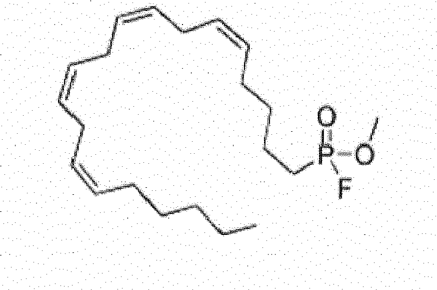 whereby MAFP (methyl-arachidonoyl-fluoro-phosphonate) is an irreversible inhibitor of cPLA2. Research has shown that this compound is also one of the most potent of the FAAH (anandamide amidohydrolase; Fatty acid amide hydrolase) inhibitors. Further studies suggest that MAFP completely blocks FAAH enzyme activity, the enzyme responsible for hydrolysis of endogenous cannabinoid ligand anandamide. Mechanistic studies suggest that this reagent specifically targets and phosphorylates arachidonyl binding sites. MAFP is an inhibitor of AChE. MAFP is an inhibitor of CB1. MAFP is an inhibitor of group VI iPLA2;
pyrrolidine-2 which is reported as having an inhibitory effect on cytosolic phospholipase A(2)alpha in non-small cell lung cancer cells (see, e.g., Sundarraj, Shenbagamoorthy; Kannan, Soundarapandian; Thangam, Ramar; et al. JOURNAL OF CANCER RESEARCH AND CLINICAL ONCOLOGY Volume: 138 Issue: 5 Pages: 827-835 Published: MAY 2012);
1-[3-(4-decoxyphenoxy)-2-oxopropyl]-3-methoxycarbonylindole-5-carboxylic acid (also referred to as CAY10502) the formula of which is as follows: 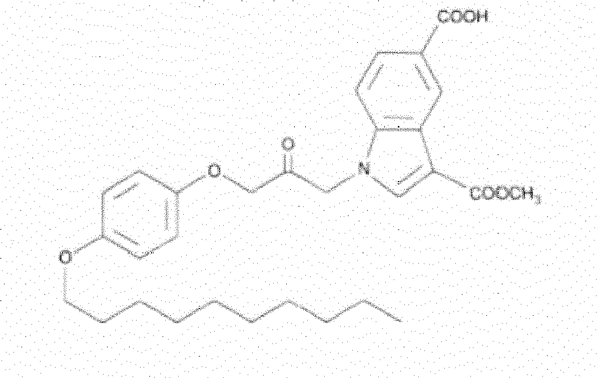 whereby CAY10502 is a potent Calcium-dependent cytosolic PLA2 (cPLA2α) inhibitor with an IC50 value of 4.3 nM for the purified enzyme from human platelets. Inhibits arachidonic acid mobilization from A23187-stimulated or TPA-stimulated human platelets with IC50 values of 570 and 0.9 nM, respectively (see, e.g., Schaloske, R.H., Dennis, E.A. The phospholipase A2 superfamily and its group numbering system. Biochem Biophys Acta 1761 1246-1259 (2006); and Ludwig, J., Bovens, S., Brauch, C., et al. Design and synthesis of 1-indol-1-yl-propan-2-ones as inhibitors of human cytosolic phospholipase A2α J Med Chem 49, 2611-2620 (2006);
pyrrophenone (N-{[(2S,4R)-1-[2-(2,4-Difluorobenzoyl)benzoyl]-4-(tritylsulfanyl)-2-pyrrolidinyl]methyl}-4-[(E)-(2,4-dioxo-1,3-thiazolidin-5-ylidene)methyl]benzamide) of the following formula 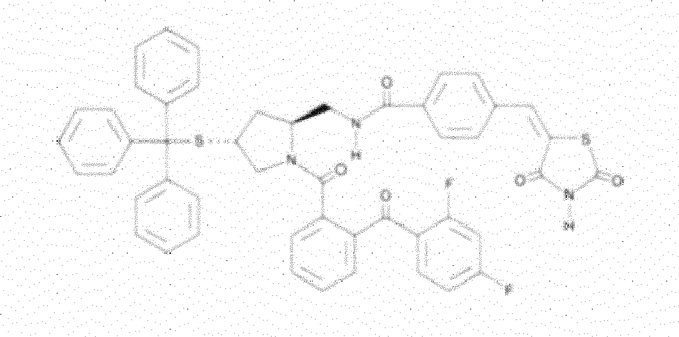 (see, e.g., Seno, K., Okuno, T., Nishi, K., et al. Pyrrolidine inhibitors of human cytosolic phospholipase A2 part2: Synthesis of potent and crystallized 4-triphenylmethylthio derivative "pyrrophenone". Bioorg Med Chem Lett 11 587-590 (2001); and Ono, T., Yamada, K., Chikazawa, Y., et al. Characterization of a novel inhibitor of cytosolic phospholipase A2α, pyrrophenone. Biochem J 363 727-735 (2002),
(E)-N-[[(2S,4R)-1-[2-(2,4-difluorobenzoyl)benzoyl]-4-[2-methylpropyl-[(2-phenylphenyl) methyl]amino]pyrrolidin-2-yl]methyl]-3-[4-[(Z)-(2,4-dioxo-1,3-thiazolidin-5-ylidene) methyl]phenyl]prop-2-enamide (also referred to as RSC-3388) of the following formula 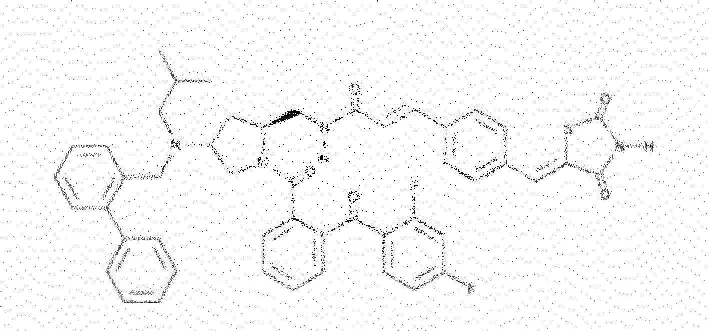 (see, e.g., Yamamoto, M., et al. 2008. Pharmacology. 81: 301-311),
quinacrine dihydrochloride dihydrate (CAS 6151-30-0, also referred to as Atabrine hydrochloride; RP-866 or SN-390) of the following formula 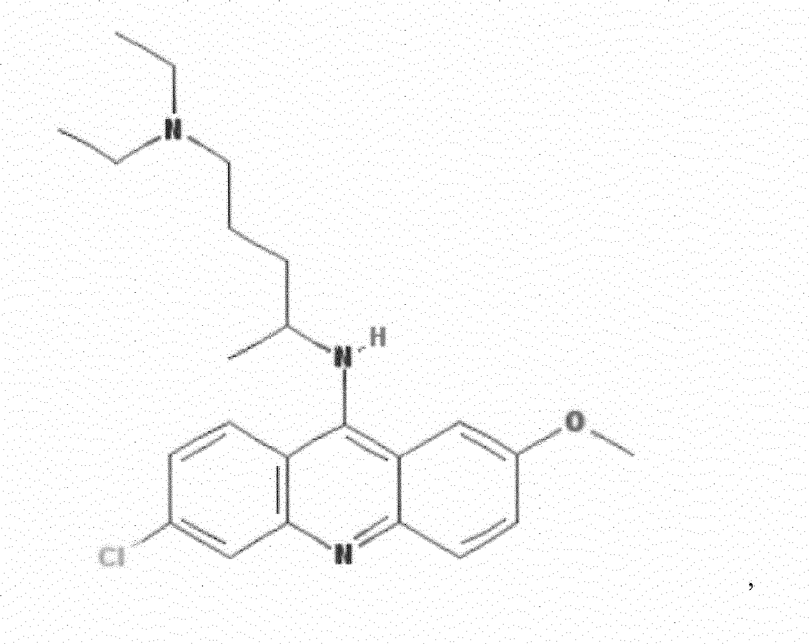 whereby quinacrine dihydrochloride dihydrate is a PLA2 (phophoslipase A2) inhibitor; it shows synergistic inhibition of prostate cancer cells when used in combination with paclitaxel or lovastatin. Quinacrine dihydrochloride dihydrate is an inhibitor of AChR, cPLA2, MAO-A and MAO-B,
an indole inhibitor of the following formula 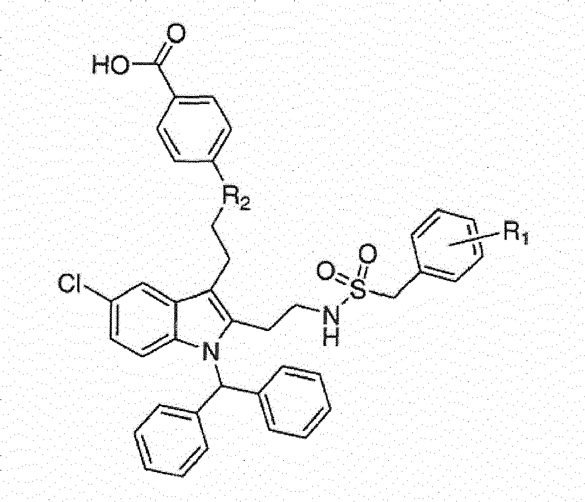 wherein R₁ is selected from the group comprising H, 3,4-diCl and 2,6-diMe, and R₂ is selected from the group comprising O and CH₂, more preferably the indole inhibitor is of the above formula, wherein R₁ is H and R₂ is O (compound 1), R₁ is 3,4-diCl and R₂ is O (compound ECOPLADIB), R₁ is 3.4-diCl and R₂ is CH2 (compound EFIPLADIB), and R₁ is 2,6-diMe and R₂ is CH2 (compound WAY196025), as described in Ramarao MK et al. (Analytical Biochemistry 383 (2008) 217-225),
a benzophenone inhibitor selected from the group comprising
compound 2b of formula 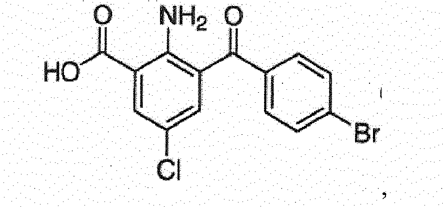 compound 2e of formula 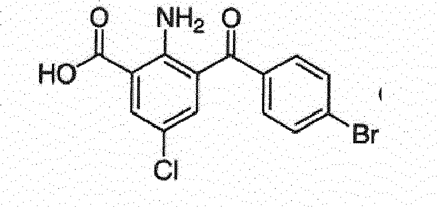 and compound 3 of formula 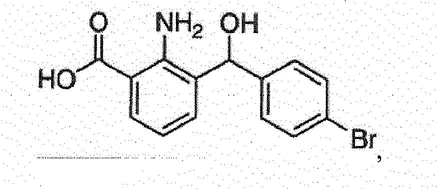 as described in Ramarao MK et al. (Analytical Biochemistry 383 (2008) 217-225),
and a pyrrolidine inhibitor selected from the group comprising
compound 4 of formula 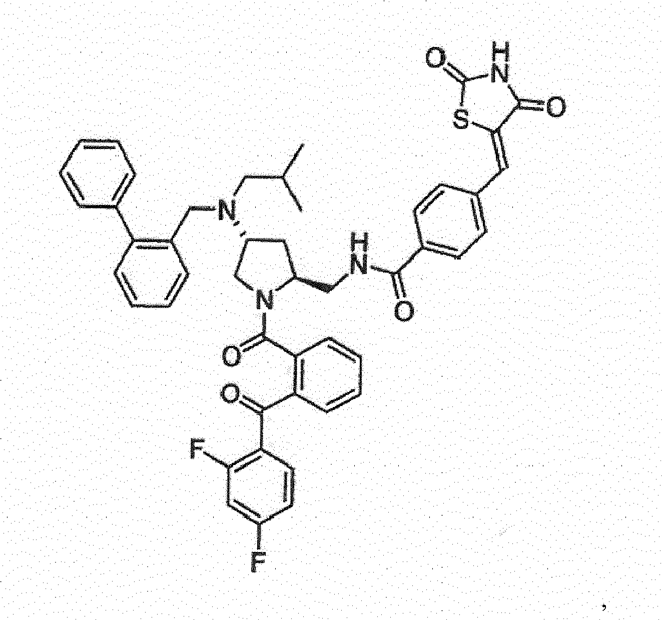 compound 5 of formula 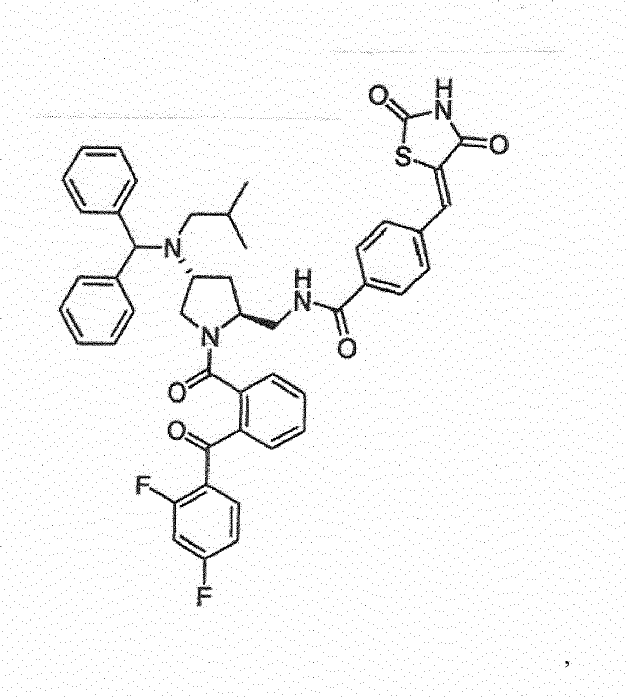 compound 6 of formula 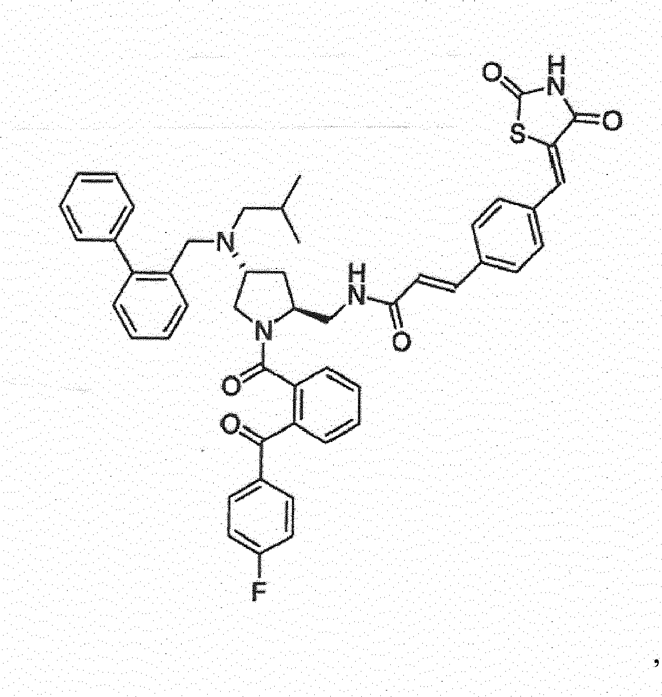 and
compound 7 of formula 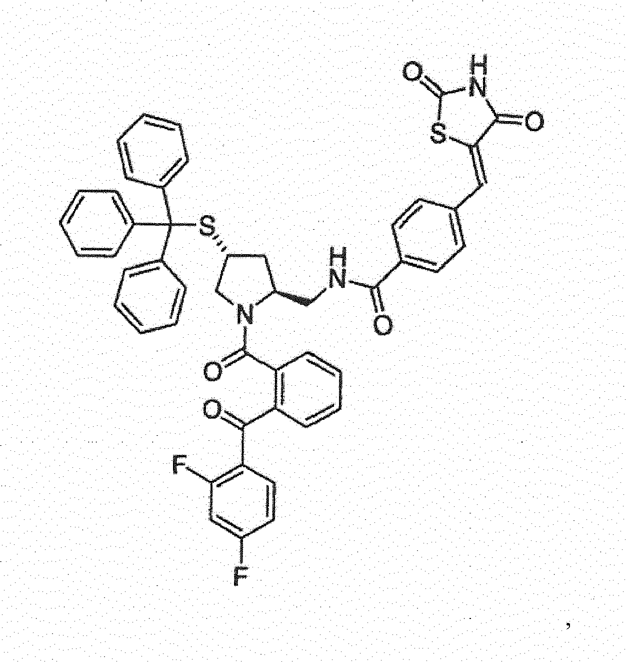 as described in Ramarao MK et al. (Analytical Biochemistry 383 (2008) 217-225).

As preferably used herein, a phenotype of cardiovascular disease is one selected from the group comprising vascular calcification, remodeling of vascular smooth muscle cells during calcification and osteochondrogenic transdifferentiation. In an embodiment thereof and as preferably used herein, remodeling of vascular smooth muscle cells is a remodeling which results in improvement, preferably clinically manifest improvement of a subject's health condition, preferably health condition. Such improved health condition preferably means that the diseases is reduced. Preferably, such reduction of disease is a clinically and/or therapeutically relevant or significant reduction. Such reduction may be expressed as reduction of vascular events, stroke, heart attack and improvement of kidney function. More preferably such improvement of kidney function is measured using one of the following parameters: urea, creatinine, Na, K, Cl, Ca, inorganic phosphor and uric acid (see, e.g., Juma AA et al. (J Clin Biochem 2012 Jan; 27(1), 40-45).

In an embodiment of the present invention, a compound which inhibits cPLA2 activity is an inhibitor of cPLA2.

It will be acknowledged by a person skilled in the art that the present invention further resides in an inhibitor of cytosolic human phospholipase A2 (cPLA2) activity such as arachidonyl trifluoromethyl ketone (AACOCF3) of formula (I) 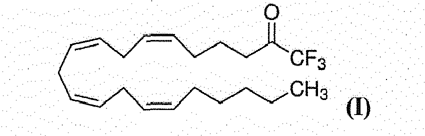 for use in the treatment of a cardiovascular disease.

Methods for determining and/or quantification of vascular calcification are known in the art and, for example, described in Voelkl J et al. (J Clin Invest. 2018; 128(7): 3024-3040) or Louvet L et al. (Nephrology Dialysis Transplantation, Volume 28, Issue 4, 1 April 2013, Pages 869-878). Basically, such method relies on Ca²⁺ measurements. for example, in such Ca²⁺ measurements, cells are washed with PBS without Ca²⁺ and Mg²⁺ and then decalcified with 0.6 N HCl overnight; the calcium content is determined colourimetrically with the o-cresolphthalein complexone method (OCP), based on the purple-coloured complex formed by calcium with OCP in an alkaline medium; the optical density of the samples is measured with a spectrophotometer at 565 nm and compared with a curve calibrated with calcium standards, alizarin red (AR) is a commonly used stain to identify calcium containing osteocytes in differentiated culture of both human and rodent mesenchymal stem cells; for AR staining, cells are washed with PBS and fixed with ethanol 95%; then, samples are exposed to AR 40 mM (pH 4.2); after two washing steps, wells are photographed showing the presence of induced mineralization. Similarly, methods for determining and/or quantifying a phenotpye of a cardiovascular disease are known in the art. For example, methods for determinig and/or quantifying osteochondrogenic transdifferentiation are based on expression of marker proteins, e.g. loss of SMC markers (SM22alpha and SM alpha-actin) and gain of osteochondrogenic markers (Runx1/Cbfa1, osteopontin, osteocalcin, and alkaline phosphatase); such methods are, for exmaple, described in Speer MY et al. (Circulation Research, vol. 104, issue 6, pp.733-U65) In a preferred embodiment, vascular calcification is one which can be treated and/or reversed by inhibiting the activtiy of PLA2, preferably cytoplasmatic PLA2.

In a clinical setting, various methods are available to assess. In general, they are based on imaging calcium content (see, e.g., Toelle M et al., Eur J Clin Invest 2015; 45(9): 976-985)

Without wishing to be bound by any theory, the present invention seems to be based on the inhibition of the activity of cytoplasmatic PLA2, also referred to as cPLA2, by arachidonyl trifluoromethyl ketone (AACOCF3) of formula (I). cPLA2 is, for example, disclosed in Kramer RM and Sharp D (FEBS Letters 410 (1997) 49-53); the involvement of cPLA2 in cardiovascular diseaes may, for example be taken from Elinder et al. (Expression of phospholipase A2 isoforms in human normal and atherosclerotic arterial wall. Arterioscler Thromb Vasc Biol. 1997 Oct;17(10):2257-63) and Hartiala et al. (Genetic contribution of the leukotriene pathway to coronary artery disease. Hum Genet. 2011 Jun;129(6):617-27). Furthermore, the effect of arachidonyl trifluoromethyl ketone (AACOCF3) of formula (I) on the treatment of a diseases associated with or caused by vascular calcification and, respectively, on reverting a molecular phenotype of a cardiovascular disease seems to be based on arachidonyl trifluoromethyl ketone (AACOCF3) of formula (I) modifying vascular changes of oxidative stress during vascular calcification and/or inhibiting migration of vascular smooth muscle cells and endothelial cells, including vascular endothelial cells. In accordance therewith, a disease in connection with each and any aspect of the present invention is also one which can be treated and/or ameliorated by the inhibition of PLA2, preferably cytoplasmatic PLA2.

In an embodiment of each and any aspect of the present invention, including any embodiment thereof, and as preferably used herein, cytoplasmic PLA2 is either isoform 1 or isoform 2, wherein preferably
isoform 1 comprises the following amino acid sequence: 
MSFIDPYQHIIVEHQYSHKFTVVVLRATKVTKGAFGDMLDTPDPYVELFISTTPDSRKRT
RHFNNDINPVWNETFEFILDPNQENVLEITLMDANYVMDETLGTATFTVSSMKVGEKKE
VPFIFNQVTEMVLEMSLEVCSCPDLRFSMALCDQEKTFRQQRKEHIRESMKKLLGPKNSE
 
GLHSARDVPVVAILGSGGGFRAMVGFSGVMKALYESGILDCATYVAGLSGSTWYMSTL
YSHPDFPEKGPEEINEELMKNVSHNPLLLLTPQKVKRYVESLWKKKSSGQPVTFTDIFGM
LIGETLIHNRMNTTLSSLKEKVNTAQCPLPLFTCLHVKPDVSELMFADWVEFSPYEIGMA
KYGTFMAPDLFGSKFFMGTVVKKYEENPLHFLMGVWGSAFSILFNRVLGVSGSQSRGST
MEEELENITTKHIVSNDSSDSDDESHEPKGTENEDAGSDYQSDNQASWIHRMIMALVSDS
ALFNTREGRAGKVHNFMLGLNLNTSYPLSPLSDFATQDSFDDDELDAAVADPDEFERIY
EPLDVKSKKIHVVDSGLTFNLPYPLILRPQRGVDLIISFDFSARPSDSSPPFKELLLAEKWA
KMNKLPFPKIDPYVFDREGLKECYVFKPKNPDMEKDCPTIIHFVLANINFRKYKAPGVPR
ETEEEKEIADFDIFDDPESPFSTFNFQYPNQAFKRLHDLMHFNTLNNIDVIKEAMVESIEY
RRQNPSRCSVSLSNVEARRFFNKEFLSKPKA (SEQ ID NO: 1) and
isoform 2 comprises the following amino acid sequence:

MSFIDPYQHIIVEHQYSHKFTVVVLRATKVTKGAFGDMLDTPDPYVELFISTTPDSRKRT
RHFNNDINPVWNETFEFILDPNQENVLEITLMDANYVMDETLGTATFTVSSMKVGEKKE
VPFIFNQVPVVAILGSGGGFRAMVGFSGVMKALYESGILDCATYVAGLSGSTWYMSTLY
SHPDFPEKGPEEINEELMKNVSHNPLLLLTPQKVKRYVESLWKKKSSGQPVTFTDIFGML
IGETLIHNRMNTTLSSLKEKVNTAQCPLPLFTCLHVKPDVSELMFADWVEFSPYEIGMAK
YGTFMAPDLFGSKFFMGTVVKKYEENPLHFLMGVWGSAFSILFNRVLGVSGSQSRGST
MEEELENITTKHIVSNDSSDSDDESHEPKGTENEDAGSDYQSDNQASWIHRMIMALVSDS
ALFNTREGRAGKVHNFMLGLNLNTSYPLSPLSDFATQDSFDDDELDAAVADPDEFERIY
EPLDVKSKKIHVVDSGLTFNLPYPLILRPQRGVDLIISFDFSARPSDSSPPFKELLLAEKWA
KMNKLPFPKIDPYVFDREGLKECYVFKPKNPDMEKDCPTIIHFVLANINFRKYKAPGVPR
ETEEEKEIADFDIFDDPESPFSTFNFQYPNQAFKRLHDLMHFNTLNNIDVIKEAMVESIEY
RRQNPSRCSVSLSNVEARRFFNKEFLSKPKA (SEQ ID NO: 2)

In connection with each and any aspect of the present invention, including any embodiment thereof, a disease is, in one embodiment, a cardiovascular disease. Preferably, the disease is one selected from the group comprising coronary artery disease (CAD), ischemic heart disease (IHD), stroke, heart failure, hypertension, angina pectoris, myocardial infarction, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis. In an embodiment, coronary artery disease (CAD) and ischemic heart disease (IHD) refer to a group of diseases comprising stable angina, unstable angina, myocardial infarction and sudden cardiac death. In a preferred embodiment, the cardiovascular disease is an atherosclerotic cardiovascular disease preferably selected from the group comprising coronary artery disease, stroke, peripheral artery disease. In a further, alternative embodiment, the disease is chronic kidney disease, preferably chronic kidney disease which is associated with and/or caused by vascular calcification, preferably vascular calcification as disclosed and/or defined herein.

In connection with each and any aspect of the present invention, including any embodiment thereof, a cholesterol lowing drug is preferably selected from the group comprising a statin, a fibrate, niacin and a bile acid sequestrant.

In connection with each and any aspect of the present invention, including any embodiment thereof, an antihypertensive drug is preferably selected from the group comprising a diuretic, a calcium channel blocker, an ACE inhibitor, an Angiotensin II receptor antagonist, an adrenergic receptor antagonist, a vasodilator, a renin inhibitor, an aldosterone receptor antagonist, an alpha-2 adrenergic receptor agonist and an endothelin receptor blocker.

In connection with each and any aspect of the present invention, including any embodiment thereof, a subject is a mammal. Preferably, the mammal is selected from the group comprising man, dog, cat, horse, cow, pig, ape, monkey, mouse and rat.

It is within the present invention that the AACOCF3 for use according to any aspect of the present invention, more specifically for use in the treatment of a subject suffering from or being at risk of suffering from a disease associated with or caused by vascular calcification; for use in therapy, wherein therapy revers a molecular phenotype of a cardiovascular disease; for use in the treatment of a cardiovascular disease; for use in the manufacture of a medicament, wherein the medicament is for use in the treatment of a subject suffering from or being at risk of suffering from a disease associated with or caused by vascular calcification; for use in the manufacture of a medicament for reverting a molecular phenotype of a cardiovascular disease; for use in the manufacture of a medicament for the treatment of a cardiovascular disease; for use in a method for treating a subject suffering from or being at risk of suffering from a disease associated with or caused by vascular calcification; for use in a method for reversing a molecular phenotype of cardiovascular disease in a subject; is present as a composition, preferably a pharmaceutical composition. Accordingly, the present invention is related in a further aspect to the use of a composition, preferably a pharmaceutical composition, comprising AACOCF3 is for use in the treatment of a subject suffering from or being at risk of suffering from a disease associated with or caused by vascular calcification; for use in therapy, wherein therapy reverts a molecular phenotype of a cardiovascular disease; for use in the treatment of a cardiovascular disease; for use in the manufacture of a medicament, wherein the medicament is for use in the treatment of a subject suffering from or being at risk of suffering from a disease associated with or caused by vascular calcification; for use in the manufacture of a medicament for reverting a molecular phenotype of a cardiovascular disease; for use in the manufacture of a medicament for the treatment of a cardiovascular disease; for use in a method for treating a subject suffering from or being at risk of suffering from a disease associated with or caused by vascular calcification; for use in a method for reversing a molecular phenotype of cardiovascular disease in a subject; or for the treatment of a vascular disease in a subject.

The synthesis of arachidonyl trifluoromethyl ketone (AACOCF3) of formula (I) 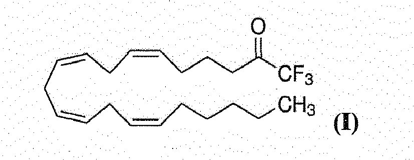 Is known to a person skilled in the art and, for example, described in Street IP et al. (Biochemistry. 1993 Jun 15; 32(23): 5935-40)
It is within the present invention that the arachidonyl trifluoromethyl ketone (AACOCF3) of formula (I) is contained in or forms part of a pharmaceutical composition, whereby such pharmaceutical composition is preferably for use in a method for treating a subject suffering from or being at risk of suffering from a disease associated with or caused by vascular calcification, for use in a method for reversing a molecular phenotype of cardiovascular disease in a subject or for the treatment of a vascular disease.

The pharmaceutical composition and the medicament, respectively, comprises, in an embodiment, at least a pharmaceutically acceptable carrier. Such carrier may be, e.g., water, buffer, PBS, glucose solution, starch, sugar, gelatine or any other acceptable carrier substance. Such carriers are generally known to the one skilled in the art. In an embodiment the carrier is ethanol of an ethanol containing aqueous solution, with the composition and medicament, respectively, preferably comprising methylcellulose in addition. In a more preferred embodiment thereof, the composition and medicament, respectively, is for oral administration.

In a further embodiment, the pharmaceutical composition and the medicament, respectively, comprises at least one second pharmaceutically active agent with the arachidonyl trifluoromethyl ketone (AACOCF3) of formula (I) being a first pharmaceutically active agent.

The preparation of a medicament and a pharmaceutical composition is known to those skilled in the art in light of the present disclosure. Typically, such composition and the medicament, respectively, may be prepared as injectables, either as liquid solutions or suspensions; as solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. A pharmaceutical composition and the medicament, respectively, may also be delivered via microdevice, microparticle or sponge.

Upon formulation, a medicament or pharmaceutical composition will be administered in a manner compatible with the dosage formulation, and in such amount as is pharmacologically effective. The formulation is easily administered in a variety of dosage forms, such as the type of injectable solutions described above, but drug release capsules and the like can also be employed.

In this context, the quantity of the pharmaceutically active agent and volume of composition to be administered depends on the individual or the subject to be treated. Specific amounts of the pharmaceutically active agent required for administration depend on the judgment of the practitioner and are peculiar to each individual.

A minimal volume of a medicament or a pharmaceutical composition required to disperse the active compounds is typically utilized. Suitable regimes for administration are also variable, but would be typified by initially administering the compound and monitoring the results and then giving further controlled doses at further intervals.

For instance, for oral administration in the form of a tablet or capsule (e.g., a gelatin capsule), the pharmaceutically active agent and/or any further pharmaceutically active agent, also referred to herein as therapeutic agent(s) or active compound(s) can be combined with an oral, non-toxic, pharmaceutically acceptable inert carrier such as ethanol, glycerol, water and the like. Moreover, when desired or necessary, suitable binders, lubricants, disintegrating agents, and coloring agents can also be incorporated into the mixture. Suitable binders include starch, magnesium aluminum silicate, starch paste, gelatin, methylcellulose, sodium carboxymethylcellulose and/or polyvinylpyrrolidone, natural sugars such as glucose or beta-lactose, corn sweeteners, natural and synthetic gums such as acacia, tragacanth or sodium alginate, polyethylene glycol, waxes, and the like. Lubricants used in these dosage forms include sodium oleate, sodium stearate, magnesium stearate, sodium benzoate, sodium acetate, sodium chloride, silica, talcum, stearic acid, its magnesium or calcium salt and/or polyethyleneglycol, and the like. Disintegrators include, without limitation, starch, methyl cellulose, agar, bentonite, xanthan gum starches, agar, alginic acid or its sodium salt, or effervescent mixtures, and the like. Diluents, include, e.g., lactose, dextrose, sucrose, mannitol, sorbitol, cellulose and/or glycine.

A pharmaceutical composition and a medicament, respectively, can also be administered in such oral dosage forms as timed release and sustained release tablets or capsules, pills, powders, granules, elixirs, tinctures, suspensions, syrups and emulsions. Suppositories are advantageously prepared from fatty emulsions or suspensions.

A pharmaceutical composition or a medicament may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating, or coating methods, and typically contain about 0.1% to 75%, preferably about 1% to 50%, of the active ingredient.

Liquid, particularly injectable compositions can, for example, be prepared by dissolving, dispersing, etc. arachidonyl trifluoromethyl ketone (AACOCF3) of formula (I) in or mixing it with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated.

For solid compositions, excipients include pharmaceutical grades of mannitol, lactose, starch, magnesium stearate, sodium saccharin, talcum, cellulose, glucose, sucrose, magnesium carbonate, and the like. The active compound defined above, may be also formulated as suppositories, using for example, polyalkylene glycols, for example, propylene glycol, as the carrier. In some embodiments, suppositories are advantageously prepared from fatty emulsions or suspensions.

A pharmaceutical composition and medicament, respectively, can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, what is well known to the ordinary skill in the art.

A pharmaceutical composition and medicament, respectively, may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, pharmaceutical composition and medicament, respectively, may be coupled to a class of biodegradable polymers useful in achieving controlled release of a drag, for example, polylactic acid, polyepsilon capro lactone, polyhydroxy butyric acid, polyorthoesters, polyacetals, polydihydropyrans, polycyanoacrylates and cross- linked or amphipathic block copolymers of hydrogels.

If desired, the pharmaceutical composition and medicament, respectively, to be administered may also contain minor amounts of non-toxic auxiliary substances such as wetting or emulsifying agents, pH buffering agents, and other substances such as for example, sodium acetate, and triethanolamine oleate.

The dosage regimen utilizing arachidonyl trifluoromethyl ketone (AACOCF3) of formula (I) and, respectively, a pharmaceutical composition or medicament comprising the same is selected in accordance with a variety of factors including type, species, age, weight, sex and medical condition of the patient; the severity of the condition to be treated; the route of administration; and the renal and hepatic function of the patient. An ordinarily skilled physician or veterinarian can readily determine and prescribe the effective amount of arachidonyl trifluoromethyl ketone (AACOCF3) of formula (I) required to prevent, counter or arrest the progress of the condition.

It will be understood that each and any embodiment disclosed herein in connection with one aspect of the present invention is also an embodiment of each and any other aspect of the present invention, including any embodiment thereof.

The present invention is further illustrated by the figures and examples from which further feature, embodiments and advantages may be taken, wherein
Fig. 1A shows four representative images of Alizarin red-stained human aortic smooth muscle cells (HAoSMCs) (n = 3) following treatment for 11 days with control or with calcification medium (Calc. Med.) without or with additional treatment with 10 µM AACOCF3; the calcified areas are shown as red staining.
Fig. 1B is a diagram indicating the activity of alkaline phosphatase (ALPL) as scatter dot plots and as arithmetic means ± SEM (n=4, U/mg protein) of alkaline phosphatase (ALPL) activity in HAoSMCs following treatment for 7 days with control or with 2 mM β-glycerophosphate (Pi) without or with additional treatment with 10 µM AACOCF3.
Figs. 1C-E are diagrams indicating relative mRNA expression in HAoSMCs of MSX2 (Fig. 1C), CBFA1 (Fig. 1D) and ALPL (Fig. 1E) as scatter dot plots and as arithmetic means ± SEM (n=6; arbitrary units, a.u.) following treatment of the HAoSMCs for 24 hours with control or with 2 mM β-glycerophosphate (Pi) without or with additional treatment with 10 µM AACOCF3.
Figs. 2A-B are diagrams indicating relative mRNA expression of NOX4 (Fig. 2A) and of CYBA (Fig. 2B) as scatter dot plots and as arithmetic means ± SEM (n=6; arbitrary units, a.u.) in HAoSMCs following treatment for 24 hours with control or with 2 mM β-glycerophosphate (Pi) without or with additional treatment with 10 µM AACOCF3.
Fig. 2C is a diagram indicating BAX/BCL2 relative mRNA expression ratio (C) in HAoSMCs following treatment for 24 hours with control or with 2 mM β-glycerophosphate (Pi) without or with additional treatment with 10 µM AACOCF3.
Fig. 3A is a diagram indicating relative mRNA expression of PLA2G4A as scatter dot plots and as arithmetic means ± SEM (n=6; arbitrary units, a.u.) in HAoSMCs following treatment for 24 hours with control or with 2mM β-glycerophosphate (Pi).
Figs 3B-D are diagram indicating relative mRNA expression of MSX2 (Fig. 3B), CBFA1 (Fig. 3C) and ALPL (Fig. 3D) in HAoSMCs following treatment for 24 hours with control or with 10 µM arachidonic acid (AA) as scatter dot plots and as arithmetic means ± SEM (n=5; arbitrary units, a.u.).
Fig. 4A is a bar diagram showing absorbance at 450 nm - as a measure of cell proliferation - for VSMCs treated with AACOCF3, with ethanol or untreated upon cultivation with 0.25 %FCS, 5 % FCS or complete medium comprising 15 % FCS.
Fig. 4B is a bar diagram indicating reduction of scratch area (µm) of VSMCs under the influence of ethanol or AACOCF3.
Fig. 4C is a panel of four representative microphotographs of VSCMs before (T0) and 18 hours after scratch (T18).
Fig. 5 is a bar diagram showing absorbance at 450 nm - as a measure of cell proliferation - for ECs treated with AACOCF3, with ethanol or untreated upon cultivation in 0.5 % FCS.
Fig. 6 is a bar diagram showing plasma lipid composition in terms of triglycerides (mmol/L), cholesterol (mmol/L), HDL (mmol/L) and LDL (mmol/L) in an animal model for atherosclerosis upon treatment with vehicle (negative control), standard therapy of Fluvastatin and Valsartan (positive control) and AACOCF3 (AUFF).
7A is a schematic illustration how the aortic root was cut so as to allow for the preparation of microsections thereof subsequently used in red oil staining.
Fig. 7B is a panel of microphotographs of various red oil stained cross sections of the aortic root upon treatment with vehicle only (vehicle), Fluvastatin and Valsartan) and AACOCF3.
Fig. 7C is a diagram indicating the quantitative relative uptake of red oil in the aortic root of animals treated with vehicle (Vehicle), standard therapy (Fluvastatin-Valsartan) or AACOCF3 (AUFF).
Fig. 8A is an image of an en face aorta which had been stained with red oil.
Figs 8B and 8C are diagrams indicating the quantitative relative uptake of red oil in total aorta (Fig. 8B) and arch of the aorta of animals treated with vehicle (Vehicle), standard therapy (Fluvastatin-Valsartan) or AACOCF3 (AUFF).
Fig. 9 is a diagram showing the relative change of collagen III per plaque upon treatment with vehicle, AACOF3 and standard therapy which is a combination of Fluvastatin and Valsartan.
Figs. 10 A - D are diagrams indicating relative mRNA expression of PLA2G4A (Fig. 10 A), MSX2 (Fig. 10 B), CBFA1 (Fig. 10 C) and ALPL (Fig 10 D) in HAoSMCs upon treatment without siRNA ("Neg. si"), cPLA2 specific siRNA ("sPLA2si"), upon treatment with phosphate but without cPLA2 specific siRNA ("Neg.si + Pi) and upon treatment with cPLA2 specific siRNA and phosphate (cPLA2si + Pi).

### Example 1: Effects of AACOCF3 on vascular smooth muscle cell calcification

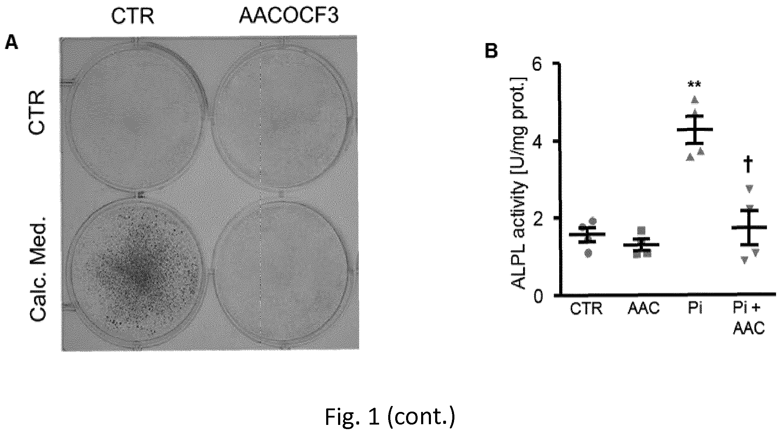

As vascular calcification is a key risk factor for cardiovascular events and mortality, the effects of the AACOCF3 compound on vascular calcification were tested. Since calcification is nearly exclusively mediated by vascular smooth muscle cells (VSMCs), experiments were performed in primary human aortic smooth muscle cells (HAoSMCs) (see, e.g., Alesutan I et al., Kidney Blood Press Res 2015;40:490-499). Calcification was induced by calcification medium containing elevated phosphate levels. In this model system a profound inhibitory effect of the AACOCF3 compound was observed during calcification as shown in Figs. 1A-E.

Primary HAoSMCs were cultured in Waymouth's MB 752/1 medium and Ham's F-12 nutrient mixture (1:1, Gibco, Life Technologies) supplemented with 10% FBS (Gibco, Life Technologies) and 100 U/ml penicillin and 100 µg/ml streptomycin. HAoSMCs were grown to confluency and used in all experiments from passages 4 to 10. Calcification was induced with 3 mM sodium phosphate buffer as calcification medium. Treatment was performed over a period of 11 days.

As evident from the results shown in more detail in the following, remodelling of VSMCs during calcification termed osteo-chondrogenic transdifferentiation, is blocked by treatment with compound AACOCF3. This is represented by impaired expression of the osteogenic transcription factors core-binding factor alpha 1 (CBFA1) / homeobox protein MSX-2 (MSX2) and the osteogenic enzyme alkaline phosphatase (ALPL) during AACOCF3 treatment in calcifying conditions.

Fig. 1A shows four representative images of Alizarin red-stained human aortic smooth muscle cells (HAoSMCs) (n = 3) following treatment for 11 days with control or with calcification medium (Calc. Med.) without or with additional treatment with 10 µM AACOCF3; the calcified areas are shown as red staining. As evident from Fig. 1A, AACOCF3 inhibits calcification of HAoSMCs.

Fig. 1B is a diagram indicating the activity of alkaline phosphatase (ALPL) as scatter dot plots and as arithmetic means ± SEM (n=4, U/mg protein) of alkaline phosphatase (ALPL) activity in HAoSMCs following treatment for 7 days with control or with 2 mM β-glycerophosphate (Pi) with Pi being a positive control inducing ALP activity, without or with additional treatment with 10 µM AACOCF3. As evident from Fig. 1B, Pi-induced ALPL activity was significantly reduced by AACOCF3 (AAC).

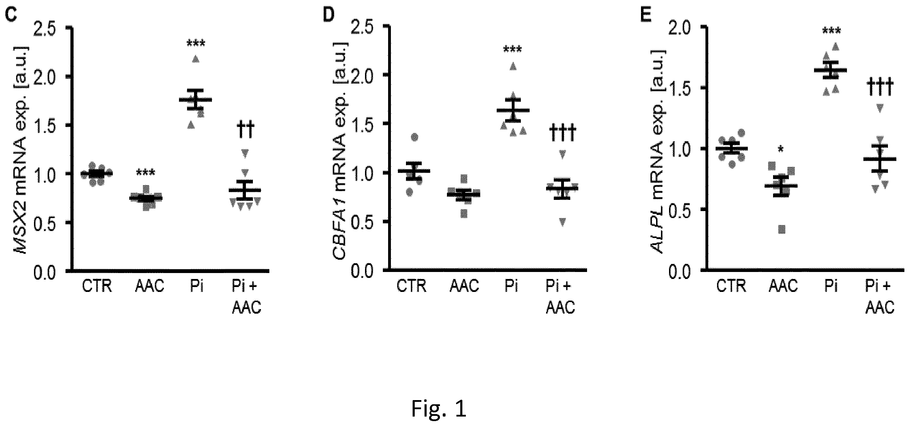

Figs. 1C-E are diagrams indicating relative mRNA expression in HAoSMCs of MSX2 (Fig. 1C), CBFA1 (Fig. 1D) and ALPL (Fig. 1E) as scatter dot plots and as arithmetic means ± SEM (n=6; arbitrary units, a.u.). mRNA expression was determined following treatment of the HAoSMCs for 24 hours with control or with 2 mM β-glycerophosphate (Pi) without or with additional treatment with 10 µM AACOCF3. **(p<0.01), ***(p<0.001) statistically significant vs. control treated HAoSMCs; †(p<0.05), ††(p<0.01), †††(p<0.001) statistically significant vs. HAoSMCs treated with Pi alone. As evident from said Figs. 1C-E, AACOCF3 was effective in significantly decreasing Pi-induced mRNA expression of MSX2, CBFA1 and ALPL.

To summarize, AACOCF3 inhibits phosphate-induced osteoinductive signaling and calcification in HAoSMCs.

### Example 2: AACOCF3 modifies vascular changes of oxidative stress during calcification

In order to assess the effect of AACOCF3 on vascular changes induced by oxidative stress during calcification, the following experiments were carried out.

Primary HAoSMCs were cultured in Waymouth's MB 752/1 medium and Ham's F-12 nutrient mixture (1:1, Gibco, Life Technologies) supplemented with 10% FBS (Gibco, Life Technologies) and 100 U/ml penicillin and 100 µg/ml streptomycin. HAoSMCs were grown to confluency and used in all experiments from passages 4 to 10.

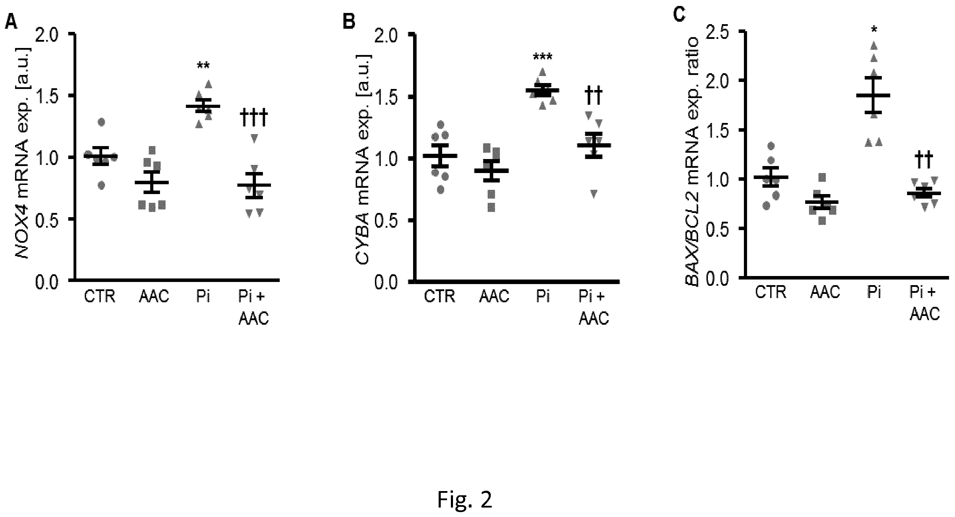

As evident from the results shown in more detail in the following, AACOCF3 significantly blunted increased expression of NADPH oxidase 4 (NOX4), cytochrome b-245 light chain (CYBA) as well as apoptosis under calcifying conditions. Such significant blunted apoptosis is illustrated and supported, respectively, by an increased expression of NADPH oxidase 4 (NOX4), cytochrome b-245 light chain (CYBA) as well as the apoptosis regulator BAX (BAX)/apoptosis regulator Bcl-2 (BCL2) ratio depicted in Figs. 2A-C.

Figs. 2A-B are diagrams indicating relative mRNA expression of NOX4 (Fig. 2A) and of CYBA (Fig. 2B) as scatter dot plots and as arithmetic means ± SEM (n=6; arbitrary units, a.u.) in HAoSMCs following treatment for 24 hours with control or with 2 mM β-glycerophosphate (Pi) without or with additional treatment with 10 µM AACOCF3. *(p<0.05), **(p<0.01), ***(p<0.001) statistically significant vs. control treated HAoSMCs; ††(p<0.01), †††(p<0.001) statistically significant vs. HAoSMCs treated with Pi alone.

Fig. 2C is a diagram indicating BAX/BCL2 relative mRNA expression ratio (C) in HAoSMCs following treatment for 24 hours with control or with 2 mM β-glycerophosphate (Pi) without or with additional treatment with 10 µM AACOCF3. *(p<0.05), **(p<0.01), ***(p<0.001) statistically significant vs. control treated HAoSMCs; ††(p<0.01), †††(p<0.001) statistically significant vs. HAoSMCs treated with Pi alone.

To summarize, AACOCF3 inhibits phosphate-inducted oxidative stress and apoptosis in HAoSMCs.

### Example 3: Mechanisms underlying the anti-calcific effect of AACOCF3

In this example, the underlying mechanisms explaining the effects of the AACOCF3 compound during calcifying conditions were addressed.

Conditions were as above (for Fig 2), namely primary HAoSMCs were cultured in Waymouth's MB 752/1 medium and Ham's F-12 nutrient mixture (1:1, Gibco, Life Technologies) supplemented with 10% FBS (Gibco, Life Technologies) and 100 U/ml penicillin and 100 µg/ml streptomycin. HAoSMCs were grown to confluency and used in all experiments from passages 4 to 10. Treatment was for 24 hours, either with control (medium only) or with 2 mM β-glycerophosphate (Pi) without or with additional treatment with 10 µM arachidonic acid (AA).

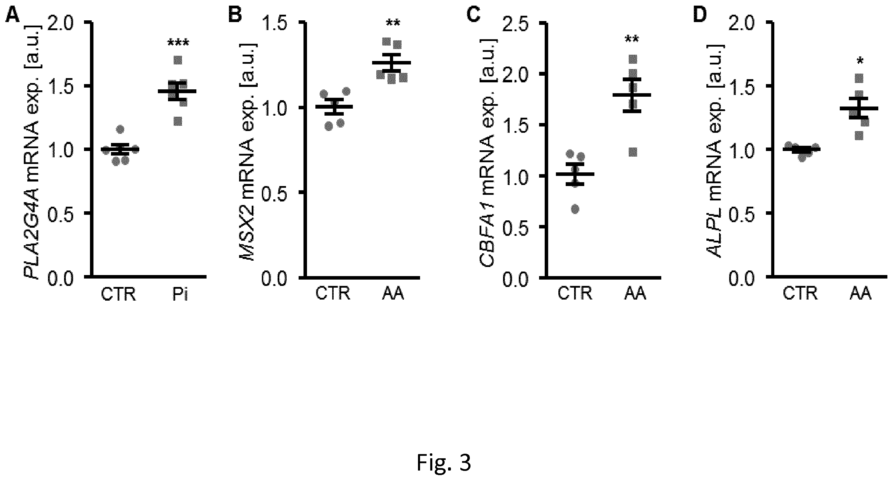

As evident from the results shown in more detail in Figs. 3A-D, calcifying conditions, i.e. phosphate treatment, induced exprssion of phospholipase A2, and osteogenic changes were replicated by treatment of vascular smooth muscle cells (VSMC) with arachidonic acid (AA).

Fig. 3A is a diagram indicating relative mRNA expression of PLA2G4A which is a specific gene, more particularly a member of the cytosolic phospholipase A2 group IV family which is also targeted by AACOCF3, as scatter dot plots and as arithmetic means ± SEM (n=6; arbitrary units, a.u.) in HAoSMCs following treatment for 24 hours with control medium or with 2mM β-glycerophosphate (Pi). ***(p<0.001) statistically significant vs. control treated HAoSMCs. PLA2G4A is a member of the cytosolic phospholipase A2 group IV family which is targeted by AACOCF3.

Figs 3B-D are diagram indicating relative mRNA expression of MSX2 (Fig. 3B), CBFA1 (Fig. 3C) and ALPL (Fig. 3D) in HAoSMCs following treatment for 24 hours with control or with 10 µM arachidonic acid (AA) as scatter dot plots and as arithmetic means ± SEM (n=5; arbitrary units, a.u.). *(p<0.05), **(p<0.01) statistically significant vs. control treated HAoSMCs.

To summarize, phosphate treatment up-regulates cytosolic phospholipase A2 expression in HAoSMCs, which results in Arachidonic acid production, which in turn induces osteoinductive signaling in HAoSMCs.

### Example 4: Effect of AACOCF3 on migration and cell proliferation

In order to assess the effect of AACOCF3 on migration and cell proliferation the following experiments were carried out. More specifically, Vascular smooth muscle cells (VSMC) and endothelial cells (EC) isolated in house from patients (human saphenous vein) were investigated to assess proliferation and migration in response to different conditions.

Vascular smooth muscle cells (VSMC) and endothelial cells (EC) isolated in house from patients (human saphenous vein) were investigated to assess proliferation and migration in response to different conditions. Vascular smooth muscle cells (VSMCs) were plated, quiesced for 72 hours and treated with 10 µM of AACOCF3 (10 µM) in the presence of medium supplemented with in 3 conditions: media with 0.2% fetal calf serum (FCS) or medium supplemented with 0.5% FCS or for VSMC and EC respectively, 5% FCS media and complete medium (smooth muscle cell media with supplements and 15% FCS). VSMC proliferation was measured by BrdU assay and migration by scratch wound assay. Endothelia cells (ECs) were plated, quiesced for 24 hours and treated with AACOCF3 (10 µM) in the presence of medium supplemented with 0.5% fetal calf serum (FCS)

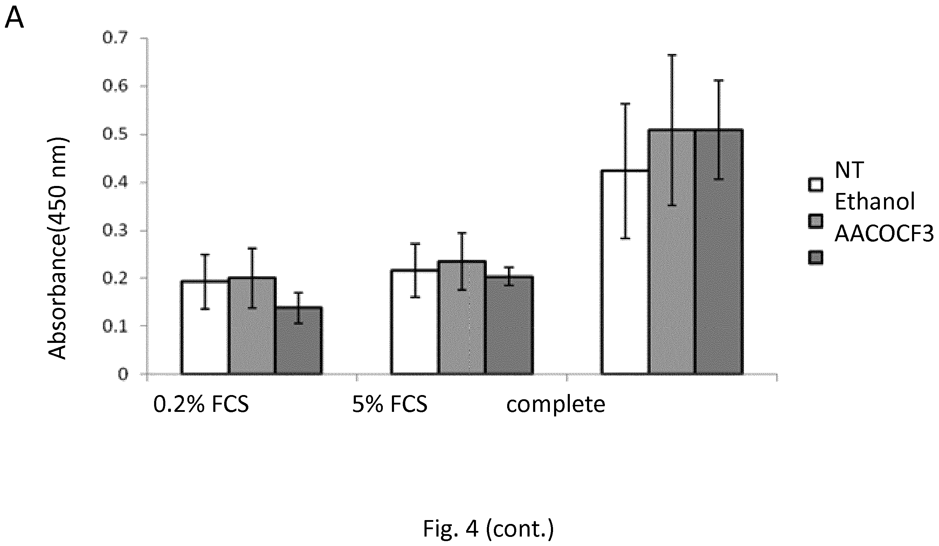

VSMC proliferation was quantified using a DNA bromodeoxyuride (BrdU) incorporation assay. The BrdU assay was assessed 48 hours after treatment with AACOCF3 (10 µM), ethanol or non-treated cells (NT). The results are shown in Fig. 4A which is a bar diagram showing absorbance at 450 nm - as a measure of cell proliferation - for VSMCs treated with AACOCF3, with ethanol or untreated upon cultivation with 0.25 %FCS, 5 % FCS or complete medium comprising 15 % FCS.

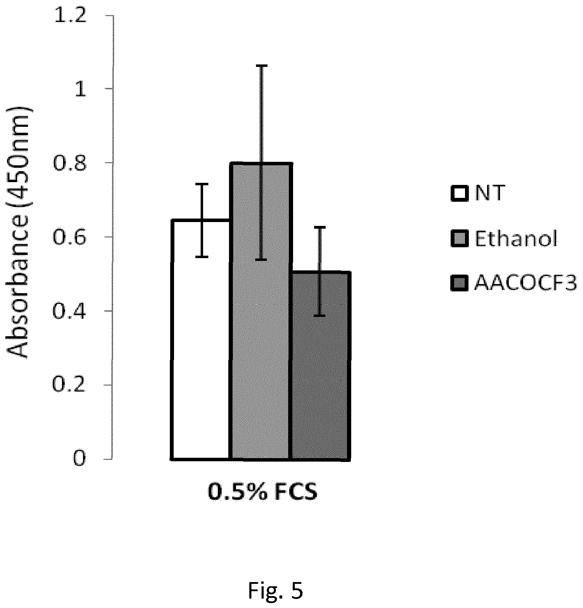

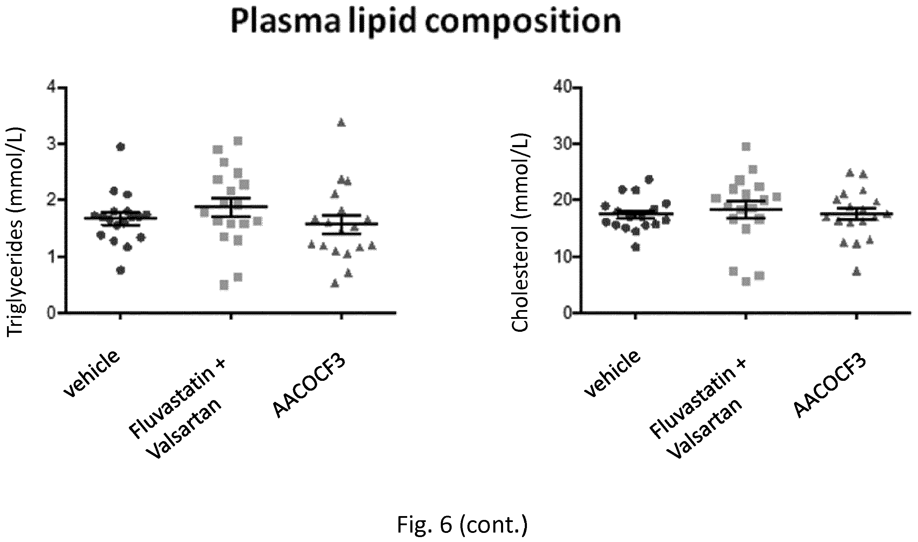

EC proliferation was quantified using a BrdU incorporation assay assessed 48 hours after treatment with AACOCF3 (10 µM), ethanol or non-treated cells (NT) (representative graph of n=2). The results are shown in Fig. 5 which is a bar diagram showing absorbance at 450 nm - as a measure of cell proliferation - for ECs treated with AACOCF3, with ethanol or untreated upon cultivation in 0.5 % FCS.

As evident from Fig. 4A, AACOCF3 did not modify proliferation of VSMC in the presence of serum, although a trend for AACOCF3 to reduce VSMC proliferation under low serum conditions was observed. Furthermore, and as shown in Fig. 5, similar observations were found for the effects of AACOCF3 on EC proliferation under similar conditions (n=2).

Additionally, AACOCF3 was also assessed for its effects on VSMC migration via scratch wound assay.

In such assay, the cell layer is wounded with a sterile 20 µL pipette tip in a predetermined grid pattern just before the treatment with AACOCF3 (10 µM) or ethanol in low serum condition. The reduction of the scratch area was measured at 18 hours after treatment (representative graph of n=2).

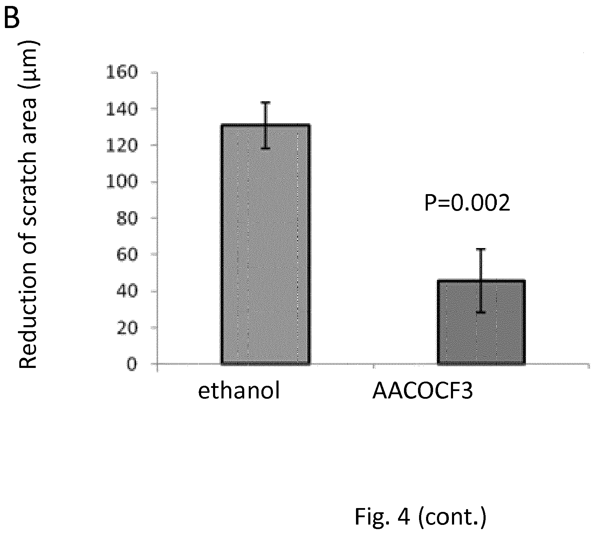

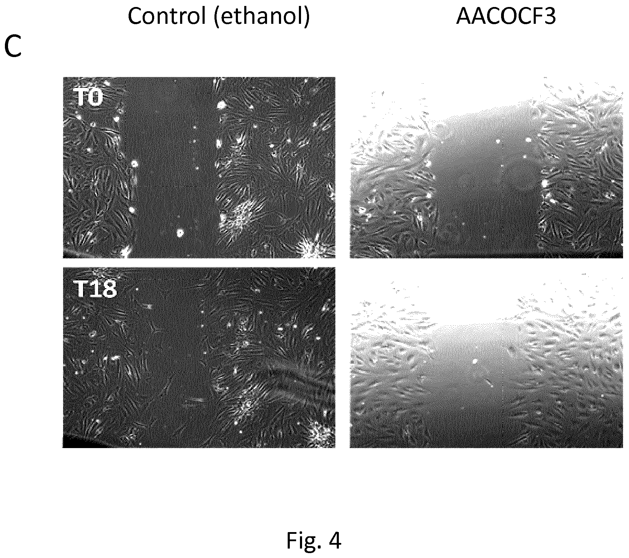

The results are shown in Figs. 4B and 4C, wherein Fig. 4B is a bar diagram indication reduction of scratch area (µm) of VSMCs under the influence of ethanol or AACOCF3 expressed as average distance between the two layers of cells measures in µm, and Fig. 4C is a panel of four representative microphotographs of VSCMs before (T0) and 18 hours after scratch (T18), whereby the wounds were photographed at baseline and 18 hours later using an EVOS XL Core camera system using a 10x objective. As evident from Figs. 4B and 4C, in two independent experiments a reduction of 67% and 65% in VSMC migration following 18 hours of AACOCF3 treatment under low serum conditions was found.

### Example 5: Effects of AACOCF3 on vascular endothelial cells (EC) proliferation.

Cells were plated, quiesced for 24 hours by starvation in 0.5% fetal calf serum (FCS) and treated with AACOCF3 (10 µM) in the presence of medium supplemented with 0.5% FCS. EC proliferation (induced by addition of 10% FCS) was quantified using a BrdU incorporation assay assessed 48 hours after treatment with AACOCF3 (10 µM), ethanol or non-treated cells (NT) (representative graph of n=2).

The above reported examples show a potential positive effect of the arachidonyl trifluoromethyl ketone (AACOCF3) of formula (I) on the cardiovascular system. To assess such positive effects further, AACOCF3 was testes in an animal model that reflects human disease to a large degree, also on the molecular level. Such animal model is the ApoE-/- mouse which was used to compare the effects of AACOCF3 treatment with standard treatment used as positive control. Such standard treatment was Fluvastatin (3 mg/kg) in combination with Valsartan (1 mg/kg) as described in Li et al. (Li et al., Hypertension 2004; 44: 758-763); AACOCF3 treatment was 10 mg/kg (AUFF); and vehicle serving as negative control was 200 µl water with 0.5 % nitrocellulose and 0.5 % ethanol; all of which were administered to the animals by gavage 5 days/week. The animals were fed with a high cholesterol diet starting at the age of 6 weeks until 16 weeks when they were sacrificed. During the study plasma und urine compositions were determined. At the end of the study, animals were euthanized, vessels were obtained and aortic lesions were assessed.

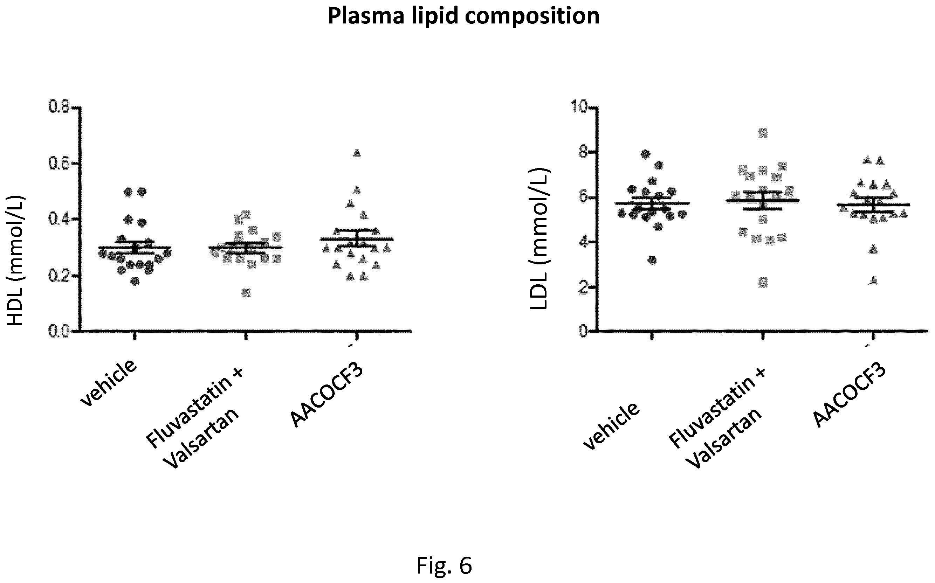

Plasma lipid composition in terms of triglycerides (mmol/L), cholesterol (mmol/L), HDL (mmol/L) and LDL (mmol/L) was determined. The results are shown in Fig. 6 for vehicle (negative control), Standard therapy of Fluvastatin and Valsartan (positive control) and AACOCF3 (AUFF).

As evident from Fig. 6, AACOCF3 treatment did not have any significant impact on plasma lipids.

Tissue from animals sacrificed at the age of 16 weeks was examined for parameters of cardiovascular disease with particular emphasis on plaque formation in the aorta and aortic root.

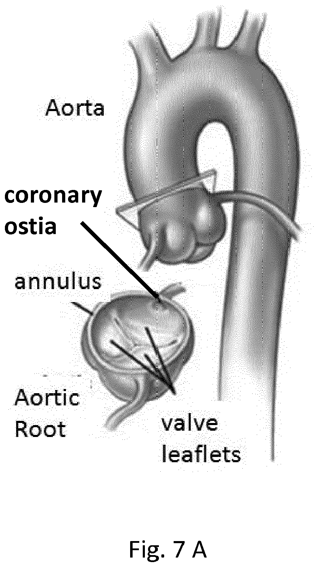

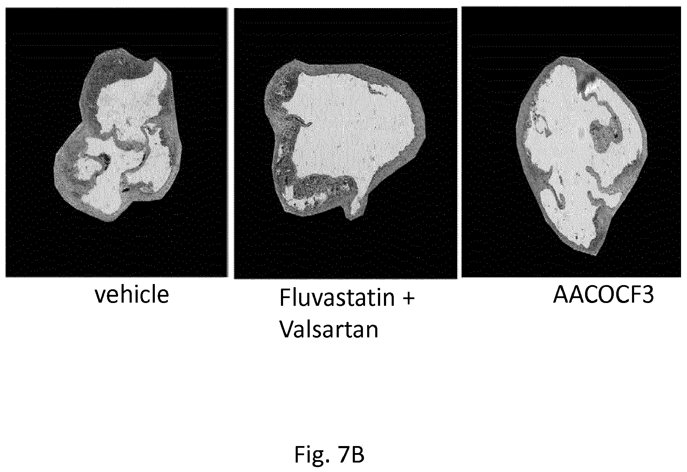

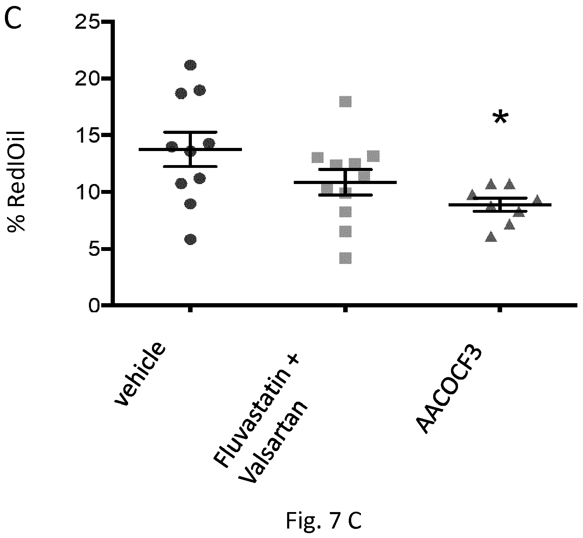

Fig. 7A is a schematic illustration how the aortic root was cut so as to allow for the preparation of microsections thereof subsequently used in red oil staining. For lipid analysis, cryo-sections were post-fixed with formaldehyde and stained with oil red O and counterstained with hematoxylin. Fig. 7B is a panel of microphotographs of sections of the aortic root after red oil staining. The thus obtained red oil stained sections of an aortic root were quantitatively analyzed in terms of relative uptake of red oil, whereby samples were imaged with a Nikon Y-FL microscope equipped with a computer-based imaging system. Staining was expressed as the percentage of total image area. The mean value of 5 sections was taken as the value for each animal. The result is shown in Fig. 7C.

As evident from both Figs. 7B and 7C, AACOCF3 prevents lipid deposition in the aortic root.

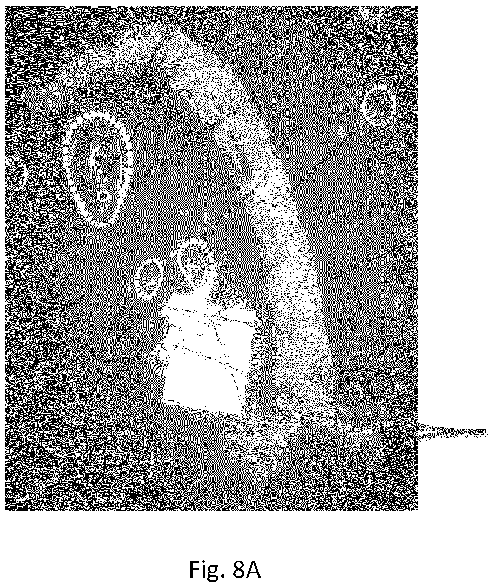

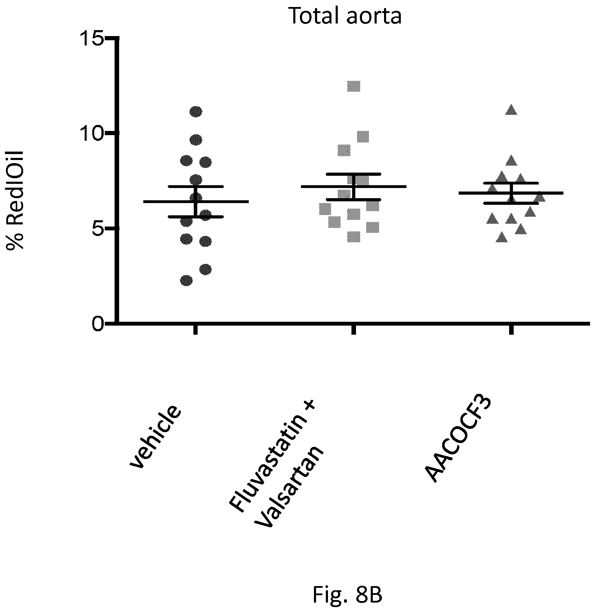

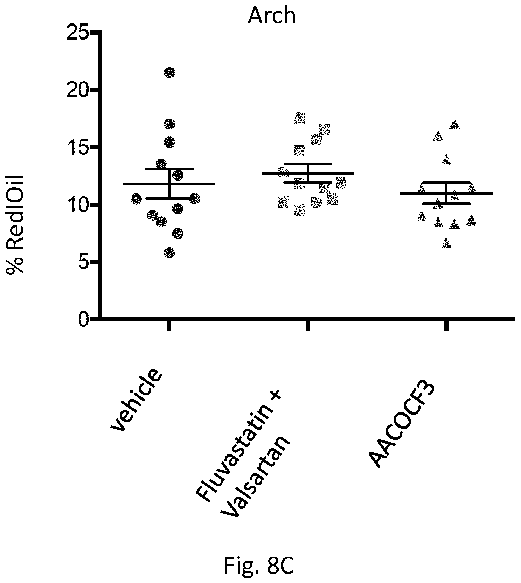

Similarly, en face aorta obtained from animals treated as outlined above was subjected to red oil staining. A photograph image of an accordingly stained en face aorta is depicted in Fig. 8A. Quantitative analysis of relative uptake of red oil was performed for all three groups of animals, i.e. animals subjected to AACOCF3 treatment, animals subjected to standard therapy (Fluvastatin -Valsartan) and animals treated with vehicle only, each for total aorta and arch of the aorta (whereby arch of the aorta refers to the section of the aorta indicated in Fig. 8A and total of the aorta refers to the remaining section of the aorta excluding the arch as indicated in Fig. 8A). The results are shown in Fig. 8B and Fig. 8C.

As evident from Figs 8B and 8C, AACOCF3 prevented lipid deposition on total aorta and on the arch of the aorta.

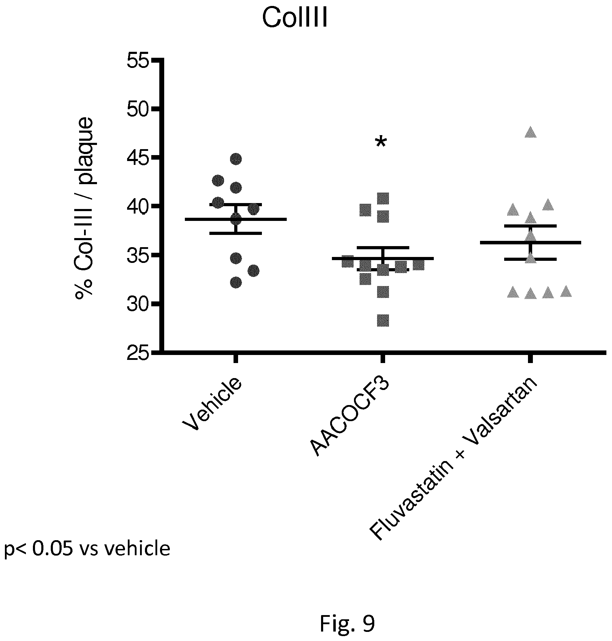

To further evaluate the changes induced in the plaques, selected representative plaques were isolated and stained for collagen type III as described: cryo-sections were post-fixed with methanol/acetate and exposed to a type III collagen primary antibody (Acris BP8014) and revealed with an anti-rabbit HRP secondary antibody (DAKO 4010). Treatment with AACOCF3 resulted in a significant reduction of the collagen type III content in the plaque, in comparison to vehicle, as shown in Figure 9.

### Example 6: Silencing of cPLA2 silencing inhibits phosphate-induced osteogenic signaling in HAoSMCs

To confirm the involvement of cPLA2 in vascular calcification, the endogenous expression of cPLA2 in HAoSMCs was suppressed by silencing of the PLA2G4A gene using siRNA. The nucleotide sequences of the double-stranded PLA2G4A.- specific siRNA were as follows:
5' UGUAUUGAGAUUCAAGCCCag 3' (SEQ ID NO: 3) (antisense strand)
5' GGGCUUGAAUCUCAAUACAtt 3' (SEQ ID NO: 4) (sense strand)

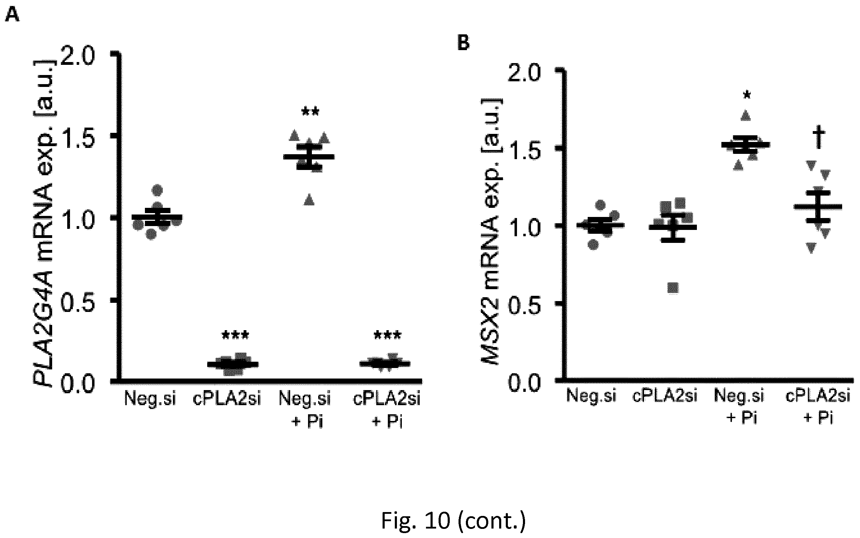

The results are shown in Figs. 10 A - D providing evidence that silencing of cPLA2 inhibited phosphate-induced osteo-/chondrogenic signaling in HAoSMCs. Scatterdot plots and arithmetic means ± SEM (n=6, arbitrary units, a.u.) of PLA2G4A (A), MSX2 (B), CBFA1 (C) and ALPL (D) relative mRNA expression in HAoSMCs following silencing with negative control siRNA (Neg.si) or cPLA2 siRNA (cPLA2si) without or with additional treatment with β-glycerophosphate (Pi). *(p<0.05), **(p<0.01), ***(p<0.001) statistically significant vs. Neg.si silenced HAoSMCs; †(p<0.05), †††(p<0.001) statistically significant vs. Neg.si silenced and Pi treated HAoSMCs.

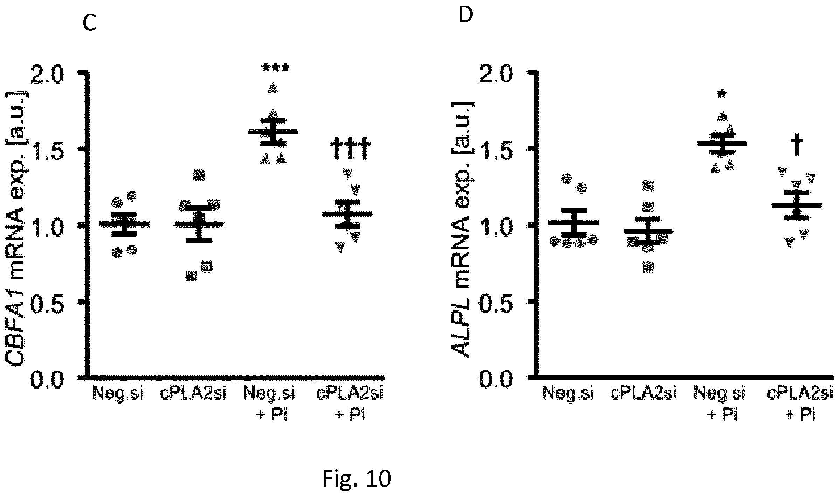

As evident from said Figs. 10 A - D, transfection with cPLA2 siRNA significantly suppressed PLA2G4A mRNA levels in HAoSMCs as compared to negative control silenced HAoSMCs (Fig. 10 A). Phosphate treatment up-regulated PLA2G4A mRNA expression in negative control silenced HAoSMCs (Fig. 10 A). Furthermore, Pi-treatment was also associated with a significant increase in cPLA2 (PLA2G4A) mRNA (Fig. 10 A). Phosphate significantly increased MSX2, CBFA1 and ALPL mRNA expression in negative control siRNA transfected HAoSMCs, effects significantly blunted in cPLA2 siRNA silenced HAoSMCs. Finally, siRNA-mediated invalidation of cPLA2a (Fig. 10 A) suppressed the elevation of Msx2, Cbfal, ALP mRNAs induced by Pi-treatment (Fig. 10 B, C and D). Taken together, the protective effects of AACOCF3 on vascular calcification involves inhibition of cPLA2-mediated AA production and release.

The features of the present invention disclosed in the specification, the claims and/or the drawings may both separately and in any combination thereof be material for realizing the invention in various forms thereof.

## Claims

1. An inhibitor of cytosolic human phospholipase A2 (cPLA2) activity for use in the treatment of a subject suffering from or being at risk of suffering from a disease caused by or associated with vascular calcification.

2. The inhibitor of cytosolic human phospholipase A2 (cPLA2) activity for use of claim 1, wherein the inhibitor is a cPLA2 inhibitor binding stoichiometrically to cPLA12 or a cPLA2 inhibitor which is a nonspecific perturbant.

3. The inhibitor of cytosolic human phospholipase A2 (cPLA2) activity for use of any one of claims 1 to 2, wherein the inhibitor is selected from the group comprising arachidonyl trifluoromethyl ketone (AACOCF3) of formula (I) 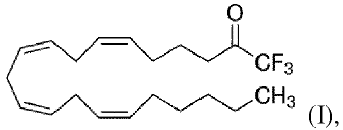 2-oxoester,
Pyrrophenone, Ecopladib, Efipladib, Giripladib, compound AX 007, compound GK 470, compounds 4 and 6 as represented by the following formulae 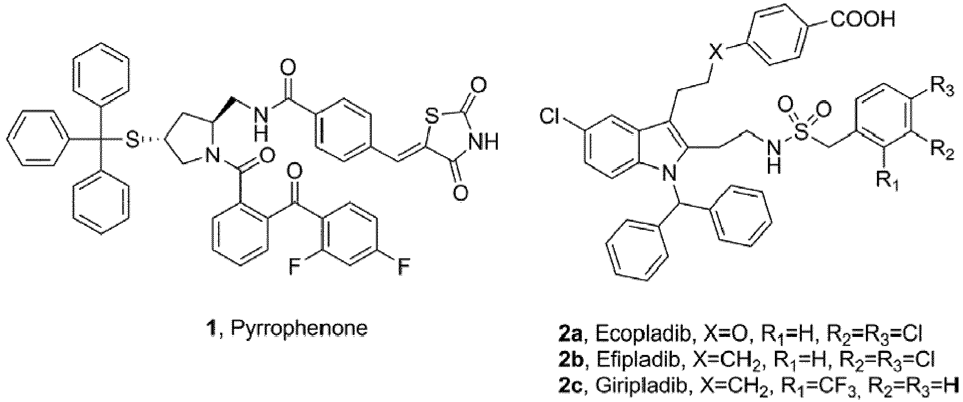 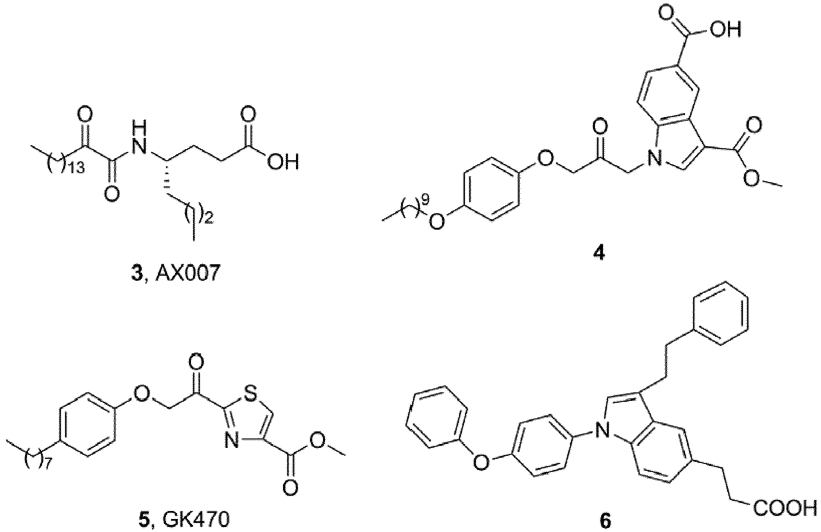 palmityl trifluoromethylketone (PTK),
Fingolimod (FTY20),
methyl arachidonyl fluorophosphonat (methylphosphonofluoridic acid 5,8,11,14-eicosatetraenyl ester),
pyrrolidine-2,
1-[3-(4-decoxyphenoxy)-2-oxopropyl]-3-methoxycarbonylindole-5-carboxylic acid, (E)-N-[[(2S,4R)-1-[2-(2,4-difluorobenzoyl)benzoyl]-4-[2-methylpropyl-[(2-phenylphenyl)methyl]amino]pyrrolidin-2-yl]methyl]-3-[4-[(Z)-(2,4-dioxo-1,3-thiazolidin-5-ylidene)methyl]phenyl]prop-2-enamide,
quinacrine dihydrochloride dihydrate (CAS 6151-30-0),
an indole inhibitor of the following formula 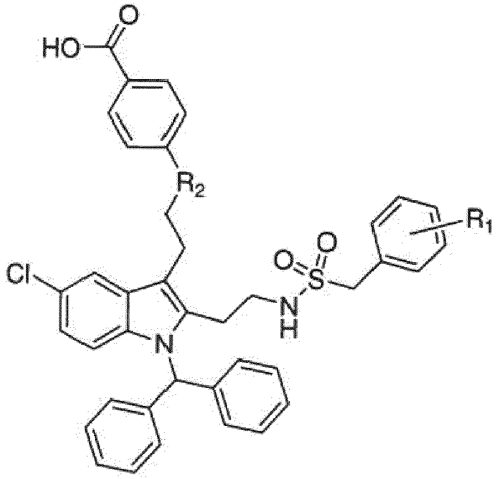 wherein R₁ is selected from the group comprising H, 3,4-diCl and 2,6-diMe, and R₂ is selected from the group comprising O and CH₂, more preferably the indole inhibitor is of the above formula, wherein R₁ is H and R₂ is O (compound 1), R₁ is 3,4-diCl and R₂ is O (compound ECOPLADIB), R₁ is 3.4-diCl and R₂ is CH2 (compound EFIPLADIB), and R₁ is 2,6-diMe and R₂ is CH2 (compound WAY196025),
a benzophenone inhibitor selected from the group comprising
compound 2b of formula 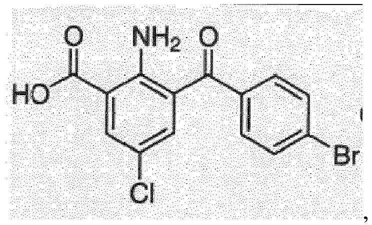 compound 2e of formula 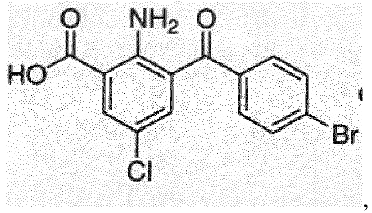 and compound 3 of formula 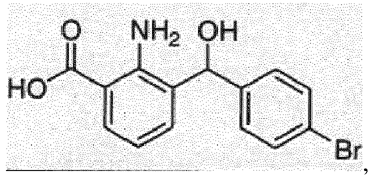 and a pyrrolidine inhibitor selected from the group comprising
compound 4 of formula 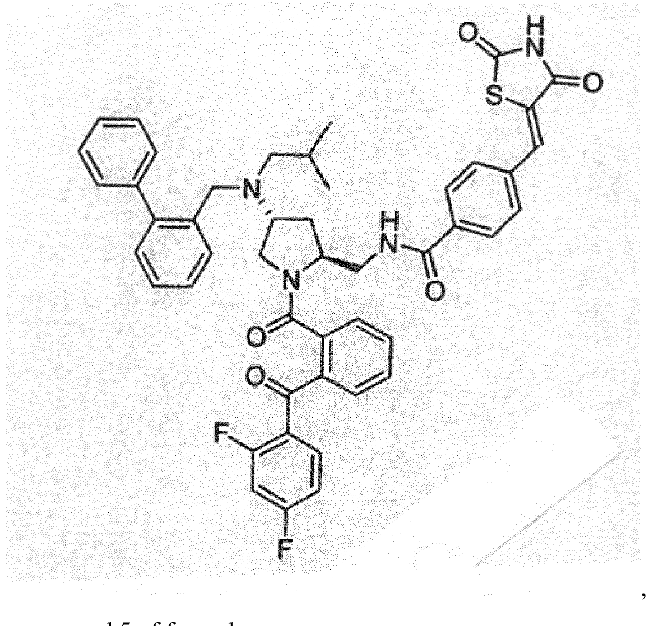 compound 5 or formula 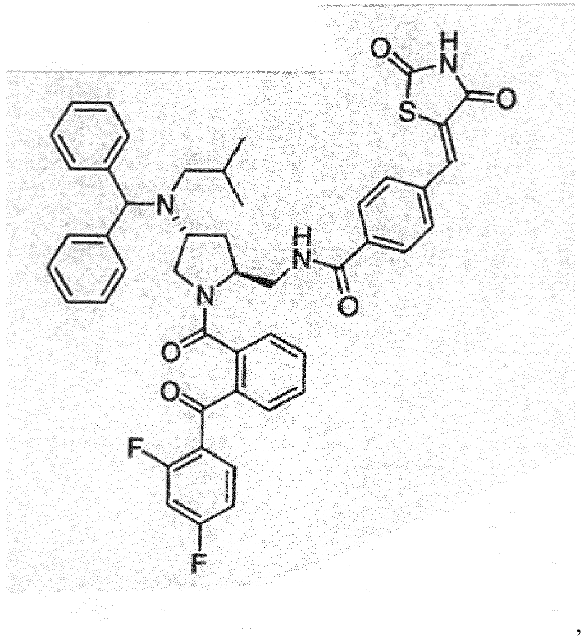 compound 6 of formula 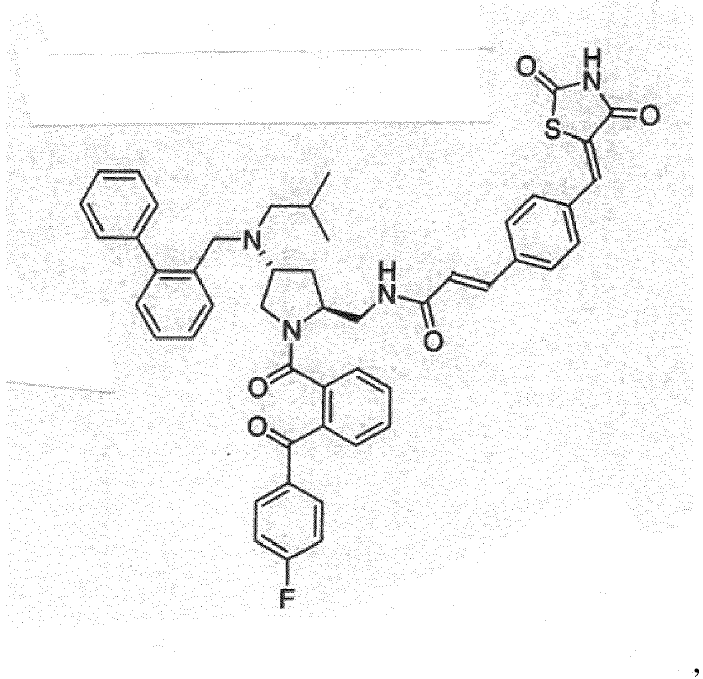 and
compound 7 of formula

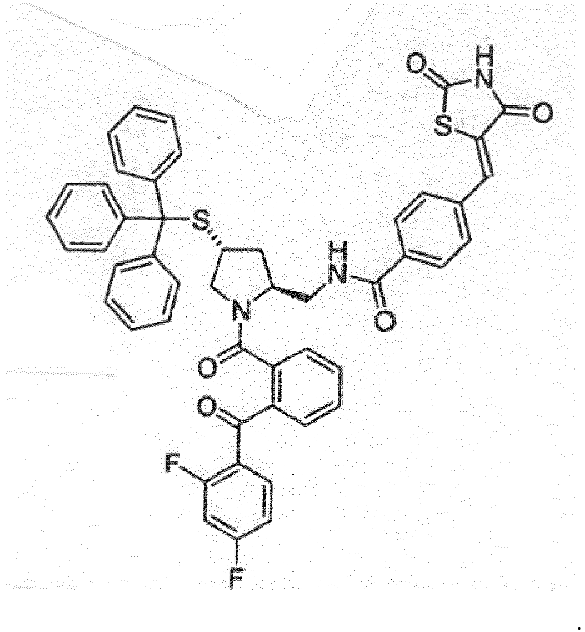

4. An arachidonyl trifluoromethyl ketone (AACOCF3) of formula (I) 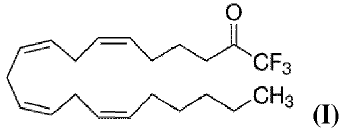 for use in the treatment of a subject suffering from or being at risk of suffering from a disease caused by or associated with vascular calcification.

5. An inhibitor of cytosolic human phospholipase A2 (cPLA2) activity for use in therapy, preferably in a therapy applied to a subject, wherein therapy reverts a molecular phenotype of a cardiovascular disease.

6. The inhibitor of cytosolic human phospholipase A2 (cPLA2) activity for use of claim 5, wherein the inhibitor is a cPLA2 inhibitor binding stoichiometrically to cPLA12 or a cPLA2 inhibitor which is a nonspecific perturbant.

7. The inhibitor of cytosolic human phospholipase A2 (cPLA2) activity for use of any one of claims 5 to 6, wherein the inhibitor is selected from the group comprising arachidonyl trifluoromethyl ketone (AACOCF3) of formula (I) 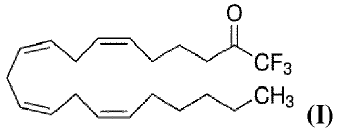 2-oxoester, Pyrrophenone, Ecopladib, Efipladib, Giripladib, compound AX 007, compound GK 470, compounds 4 and 6 as represented by the following formulae 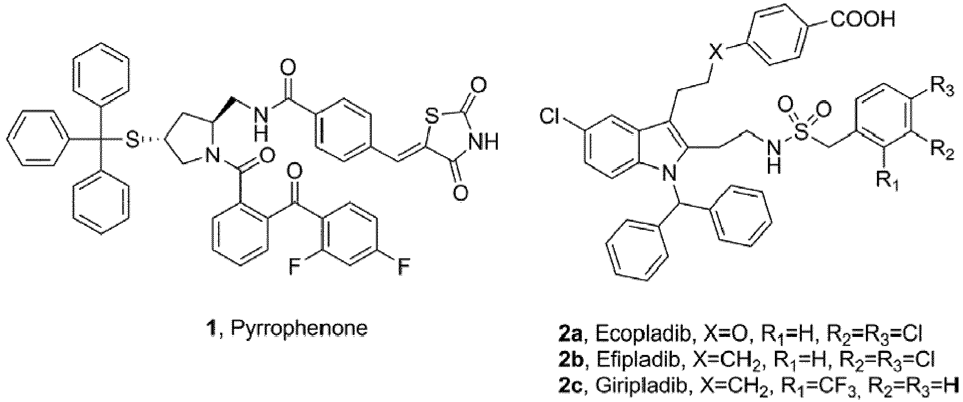 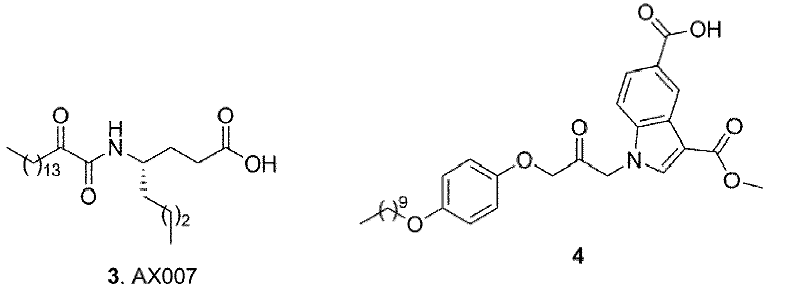 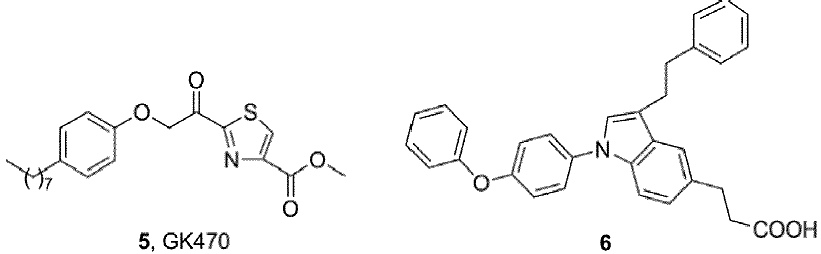 palmityl trifluoromethylketone (PTK),
Fingolimod (FTY20),
methyl arachidonyl fluorophosphonat (methylphosphonofluoridic acid 5,8,11,14-eicosatetraenyl ester),
pyrrolidine-2,
1-[3-(4-decoxyphenoxy)-2-oxopropyl]-3-methoxycarbonylindole-5-carboxylic acid, (E)-N-[[(2S,4R)-1-[2-(2,4-difluorobenzoyl)benzoyl]-4-[2-methylpropyl-[(2-phenylphenyl)methyl]amino]pyrrolidin-2-yl]methyl]-3-[4-[(Z)-(2,4-dioxo-1,3-thiazolidin-5-ylidene)methyl]phenyl]prop-2-enamide,
quinacrine dihydrochloride dihydrate (CAS 6151-30-0),
an indole inhibitor of the following formula 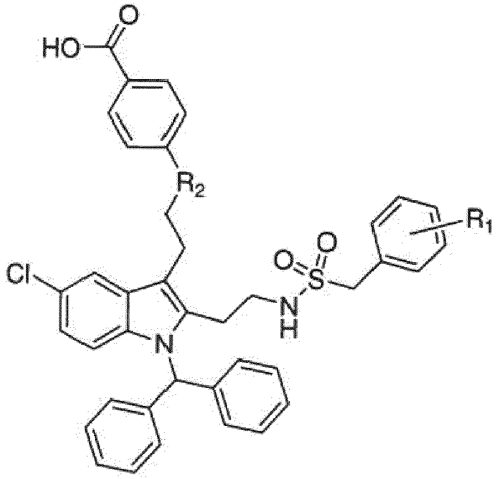 wherein R₁ is selected from the group comprising H, 3,4-diCl and 2,6-diMe, and R₂ is selected from the group comprising O and CH₂, more preferably the indole inhibitor is of the above formula, wherein R₁ is H and R₂ is O (compound 1), R₁ is 3,4-diCl and R₂ is O (compound ECOPLADIB), R₁ is 3.4-diCl and R₂ is CH2 (compound EFIPLADIB), and R₁ is 2,6-diMe and R₂ is CH2 (compound WAY196025),
a benzophenone inhibitor selected from the group comprising
compound 2b of formula 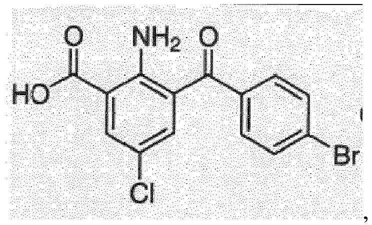 compound 2e of formula 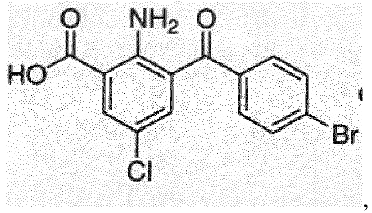 and compound 3 of formula 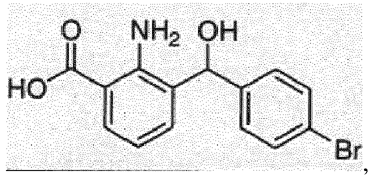 and a pyrrolidine inhibitor selected from the group comprising
compound 4 of formula 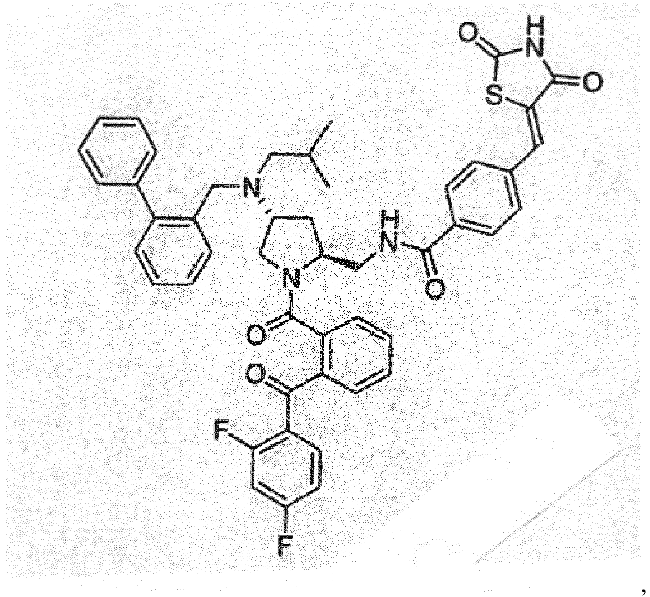 compound 5 of formula 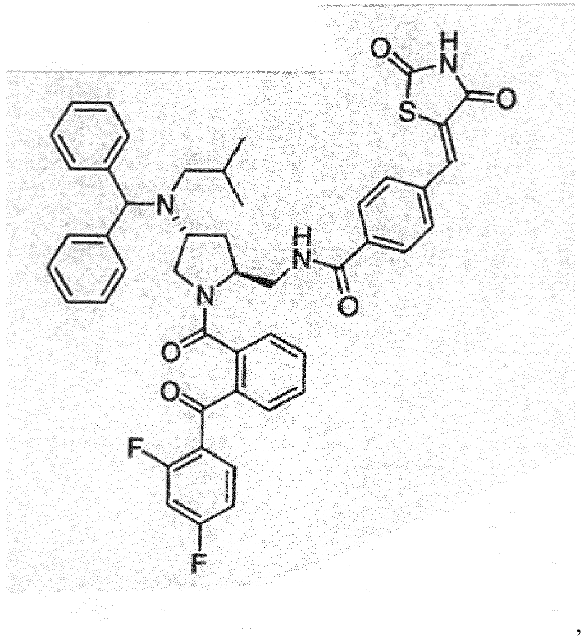 compound 6 of formula 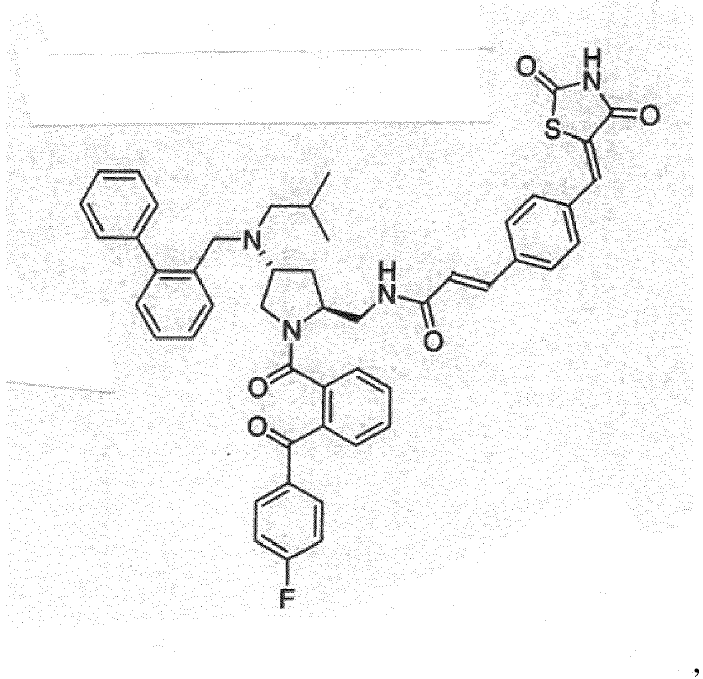 and compound 7 of formula

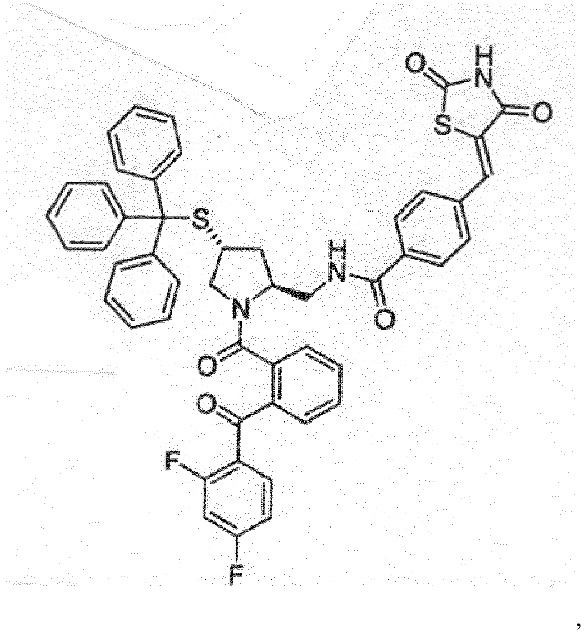

8. An arachidonyl trifluoromethyl ketone (AACOCF3) of formula (I) 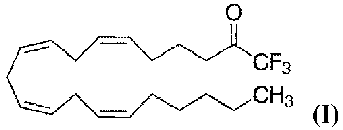 for use in therapy, preferably in a therapy applied to a subject, wherein therapy reverts a molecular phenotype of a cardiovascular disease.

9. The inhibitor of cytosolic human phospholipase A2 (cPLA2) activity for use of any one of claims 1 to 4, wherein vascular calcification is mediated by vascular smooth muscle cells.

10. The inhibitor of cytosolic human phospholipase A2 (cPLA2) activity for use of claim 9, wherein vascular smooth muscle cells are re-modelled during calcification.

11. The inhibitor of cytosolic human phospholipase A2 (cPLA2) activity for use of any one of claims 1 to 4 and 9 to 10, wherein vascular calcification results from osteochondrogenic transdifferentiation.

12. The arachidonyl trifluoromethyl ketone (AACOCF3) of formula (I) for use of any one of claims 4 to 8, wherein AACOCF3 inhibits cPLA2 activity.

13. The arachidonyl trifluoromethyl ketone (AACOCF3) of formula (I) for use of any one of claims 1 to 4 and 9 to 12, wherein the disease is a cardiovascular disease or a chronic kidney disease.

14. The inhibitor of cytosolic human phospholipase A2 (cPLA2) activity for use in any one of claims 5 to 8 and 13, wherein the cardiovascular disease is selected from the list comprising CAD, stroke, heart failure, hypertension, angina pectoris, myocardial infarction, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis, preferably the cardiovascular disease is an atherosclerotic cardiovascular disease preferably selected from the group comprising coronary artery disease, stroke, peripheral artery disease, and.

15. The inhibitor of cytosolic human phospholipase A2 (cPLA2) activity for use of any one of claims 1 to 14, wherein the subject shows at least one cardiovascular disease risk factor selected from the group comprising little exercise, unhealthy nutrition, smoking and drinking.

16. The inhibitor of cytosolic human phospholipase A2 (cPLA2) activity for use of any one of claims 1 to 15, wherein the subject has a risk factor selected from the group comprising smoking, diabetes, hypertension and dyslipidaemia.

17. The inhibitor of cytosolic human phospholipase A2 (cPLA2) activity for use of any one of claims 1 to 16, wherein the treatment further comprises a therapy selected from the group comprising a cholesterol lowering drug, an antihypertensive drug and surgical intervention, preferably coronary artery bypass grafting (CABG), percutaneous coronary intervention (PCI) and stenting of vasculature.

18. The inhibitor of cytosolic human phospholipase A2 (cPLA2) activity for use of claim 17, wherein the cholesterol lowering drug is selected from the group comprising a statin, a fibrate, niacin, and a bile acid sequestrant.

19. The inhibitor of cytosolic human phospholipase A2 (cPLA2) activity for use of claim 17, wherein the antihypertensive drug is selected from the group comprising a diuretic, a calcium channel blocker, an ACE inhibitor, an angiotensin II receptor antagonist, an adrenergic receptor antagonist, a vasodilator, a renin inhibitor, an aldosterone receptor antagonist, an alpha-2 adrenergic receptor agonist, and an endothelin receptor blocker.
